(19) Europäisches Patentamt
European Patent Office
Office européen des brevets

(11) EP 3 888 655 B1

# (12) EUROPEAN PATENT SPECIFICATION

(45) Date of publication and mention of the grant of the patent:
**30.08.2023 Bulletin 2023/35**

(21) Application number: **19889734.0**

(22) Date of filing: **28.11.2019**

(51) International Patent Classification (IPC):
*A61K 31/496* (2006.01) *A61K 31/5415* (2006.01)
*A61K 31/395* (2006.01) *A61K 31/437* (2006.01)
*A61K 31/438* (2006.01) *A61K 31/538* (2006.01)
*A23L 33/10* (2016.01) *A61P 25/28* (2006.01)

(52) Cooperative Patent Classification (CPC):
**A61K 31/5415; A23L 33/10; A61K 31/395; A61K 31/437; A61K 31/438; A61K 31/496; A61K 31/538; A61P 25/28**

(86) International application number:
**PCT/KR2019/016599**

(87) International publication number:
**WO 2020/111824 (04.06.2020 Gazette 2020/23)**

## (54) THERAPEUTIC COMPOSITION COMPRISING RIFAPENTINE FOR USE IN TREATING AMYLOID NEUROLOGICAL DISEASES

THERAPEUTISCHE ZUSAMMENSETZUNG ENTHALTEND RIFAPENTIN ZUR VERWENDUNG BEI DER BAHANDLUNG VON AMYLOID-NEUROLOGISCHEN ERKRANKUNGEN

COMPOSITION THÉRAPEUTIQUE COMPRENANT LA RIFAPENTINE POUR UTILISATION DANS LE TRAITEMENT DE MALADIES NEUROLOGIQUES AMYLOÏDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.11.2018 KR 20180151835**
**12.12.2018 KR 20180160289**

(43) Date of publication of application:
**06.10.2021 Bulletin 2021/40**

(73) Proprietor: **Amyloid Solution Inc.**
**Gyeonggi-do 13488 (KR)**

(72) Inventors:
- **KIM, Seong Muk**
  **Seoul 07651 (KR)**
- **KIM, Young Soo**
  **Incheon 21982 (KR)**
- **SHIN, Ji Su**
  **Incheon 21022 (KR)**
- **JEON, Han Na**
  **Gwangju-si, Gyeonggi-do 12770 (KR)**
- **LEE, Dong Hee**
  **Incheon 22705 (KR)**
- **KIM, Hye Ju**
  **Hwaseong-si, Gyeonggi-do 18444 (KR)**

(74) Representative: **Vossius & Partner**
**Patentanwälte Rechtsanwälte mbB**
**Siebertstraße 3**
**81675 München (DE)**

(56) References cited:
**EP-A1- 0 675 126 EP-A1- 3 650 024**
**WO-A1-95/11248 WO-A1-2017/003469**

- **LUCAS A R: "Gilles de la Tourette's disease in children: Treatment with phenothiazine drugs", AMERICAN JOURNAL OF PSYCHIATRY 1964, vol. 121, no. 6, 1964, pages 606-608, XP009533059, ISSN: 0002-953X**
- **TOMIYAMA, T. et al.: "Rifampicin prevents the aggregation and neurotoxicity of amyloid beta protein in vitro", Biochemical and Biophysical Research Communications, vol. 204, no. 1, 1994, pages 76-83, XP024765855, DOI: 10.1006/bbrc.1994.2428**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- UMEDA, T. et al.: "Rifampicin is a candidate preventive medicine against amyioid-beta and tau oligomers", Brain., vol. 139, no. 5, 2016, pages 1568-1586, XP055658105,
- UMEDA, T. et al.: "Intranasal rifampicin for Alzheimer's disease prevention", Alzheimer's & Dementia: Translational Research & Clinical Interventions, vol. 4, 14 July 2018 (2018-07-14), pages 304-313, XP055713181,
- IIZUKA, T. et al.: "Preventive Effect of Rifampicin on Alzheimer Disease Needs at Least 450 mg Daily for 1 Year: An FDG-PET Follow-Up Study", Dement. Geriatr. Cogn. Dis. Extra., vol. 7, no. 2, 2017, pages 204-214, XP055713182,

## Description

TECHNICAL FIELD

The present disclosure relates to a composition comprising rifapentine for use in the treatment of amyloid cranial neuropathy, in particular for use in preventing or treating neurodegenerative brain disease, as defined in the claims.

BACKGROUND ART

Neurodegenerative brain diseases are diseases that cause degenerative changes in nerve cells of the central nervous system and thus leads to various symptoms such as impaired motor and sensory functions, inhibition of high-dimensional functions such as memory, learning, and calculation. Common neurodegenerative brain diseases include Alzheimer's disease, Parkinson's disease, and memory disorders. In neurodegenerative brain diseases, neuronal cell death occurs due to rapid or slow progression of necrosis or apoptosis. Therefore, for the development of a method of preventing, controlling, and treating central nervous system diseases, the mechanism of death of neurons needs to be understood.

Meanwhile, an important pathological feature of Alzheimer's, a common neurodegenerative brain disease, is the formation of peptide aggregates called senile plaques, which cause synaptic dysfunction and neuronal death. The main component of these senile plaques is amyloid-beta having a length of 40-42 amino acids. Amyloid-beta monomers easily self-assemble into oligomers, protofibrils, and beta-sheet-rich fibrils, and are associated with the development of neurotoxicity.

The correlation between amyloid-beta plaques and neurotoxicity has not yet been identified. However, the self-assembly of amyloid-beta into intermediate oligomers or aggregates is thought to be associated with the development of neurological diseases such as Alzheimer's disease.

The Tau protein consists of four parts including the N-terminal overhang, the proline aggregation domain, the microorganism binding domain and the C-terminal (Mandelkow et al., Acta.Neuropathol., 103, 26-35, 1996), and abnormal hyperphosphorylation or deformation of Tau in neurons of the central nervous system is known to cause neurodegenerative brain diseases such as Parkinson's disease and Tauopathy.

Therefore, the inhibition of amyloid-beta aggregation, the degradation of amyloid-beta aggregates, or the reduction of abnormally hyperphosphorylated Tau proteins has been suggested as a method of treating neurodegenerative brain diseases such as Alzheimer's and Parkinson's disease.

EP-A-0675126 describes a beta-amyloid protein aggregation and/or deposition inhibitor which contains a rifamycin for use in treating or preventing senile dementia of the Alzheimer type. Tomiyama et al. disclose in Biochem. Biophys. Res. Comm., 204(1), 1994, 76-83, that rifampicin may be a potential therapeutic agent for inhibiting the initial step of amyloid formation in Alzheimer's disease. Umeda et al. disclose in Brain, 139(5), 2016, 1568-1586, that rifampicin could be a promising, ready-to-use medicine for the prevention of Alzheimer's disease and other neurodegenerative diseases. Umeda et al. describe in Alzheimer's & Dementia: Translational Research & Clinical Interventions, 4, 2018, 304-313 the use of rifampicin for the prevention of Alzheimer's disease. Izuka et al. report in Dement. Geriatr. Cogn. Dis. Extra., 7(2), 2017, 204-214 that rifampicin has preventive effects on Alzheimer's disease and cognitive decline in humans.

DESCRIPTION OF EMBODIMENTS

TECHNICAL PROBLEM

The references to methods of treatment in this description are to be interpreted as references to the compounds, pharmaceutical compositions and medicaments of the present invention for use in a method for treatment of the human (or animal) body by therapy. A non-claimed aspect provides a pharmaceutical composition for the inhibition and/or degration of amyloid-beta aggregation, the pharmaceutical composition including, as an active ingredient, one or more selected from the group consisting of rifamycin, triflupromazine, and pharmaceutically acceptable salts thereof.

Another non-claimed aspect provides a pharmaceutical composition for the degradation of Tau proteins (and/or inhibition of Tau proteins aggregation) and/or inhibition of phosphorylation of Tau proteins, the pharmaceutical composition including, as an active ingredient, one or more selected from the group consisting of rifamycin, triflupromazine, and pharmaceutically acceptable salts thereof.

Another non-claimed aspect provides a method of inhibiting and/or degrading amyloid-beta aggregation degrading, the method including administering, to a subject in need of amyloid-beta aggregation inhibition and/or degradation, a pharmaceutically effective amount of one or more selected from the group consisting of rifamycin, triflupromazine, and pharmaceutically acceptable salts thereof. The method may further include, before the administering,

identifying a subject in need of the inhibition and/or degradation of amyloid-beta aggregation.

Another non-claimed aspect provides a method of degrading (and/or inhibiting of aggregation) Tau proteins and/or inhibiting phosphorylation of Tau proteins, the method including administering, to a subject in need of degradation (and/or inhibition of aggregation) of Tau proteins and/or inhibition of phosphorylation of Tau proteins, a pharmaceutically effective amount of one or more selected from the group consisting of rifamycin, triflupromazine, and pharmaceutically acceptable salts thereof. The method may further include, before the administering, identifying a subject in need of degradation (and/or inhibition of aggregation) of Tau proteins and/or the inhibition of phosphorylation of Tau proteins.

An embodiment of the invention provides a pharmaceutical composition for use in the prevention and/or treatment of neurodegenerative brain disease, the pharmaceutical composition including, as an active ingredient, rifapentine or pharmaceutically acceptable salts thereof.

Another non-claimed aspect provides a method of preventing and/or treating neurodegenerative brain disease, the method including administering, to a subject in need of the prevention and/or treatment of neurodegenerative brain disease, a pharmaceutically effective amount of one or more selected from the group consisting of rifamycin, triflupromazine, and pharmaceutically acceptable salts thereof. The method may further include, before the administering, identifying a subject in need of the prevention and/or treatment of neurodegenerative brain disease.

Another non-claimed aspect provides a pharmaceutical composition for neuroprotection, the pharmaceutical composition including, as an active ingredient, one or more selected from the group consisting of rifamycin, triflupromazine, and pharmaceutically acceptable salts thereof.

Another non-claimed aspect provides a method of protecting cranial neuronal cells, the method including administering, to a subject in need of protection of cranial neuronal cells, a pharmaceutically effective amount of one or more selected from the group consisting of rifamycin, triflupromazine, and pharmaceutically acceptable salts thereof. The method may further include, before the administering, identifying a subject in need of the protection of cranial neuronal cells.

Another embodiment provides a pharmaceutical composition for use in the prevention or amelioration of cognitive disorders or the therapeutic improvement of memory, the pharmaceutical composition including, as an active ingredient, rifapentine or pharmaceutically acceptable salts thereof.

Another non-claimed aspect provides a method of preventing or ameliorating cognitive disorders or improving memory, the method including administering, to a subject in need of preventing or ameliorating cognitive disorders or improving memory, a pharmaceutically effective amount of one or more selected from the group consisting of rifamycin, triflupromazine, and pharmaceutically acceptable salts thereof. The method may further include, before the administering, identifying a subject in need of the prevention or amelioration of cognitive disorders or the improvement of memory.

Another non-claimed aspect provides a health functional food for inhibiting amyloid-beta aggregation and/or degrading amyloid-beta, the health functional food including, as an active ingredient, one or more selected from the group consisting of rifamycin, triflupromazine, and sitologically acceptable salt thereof.

Another non-claimed aspect provides a health functional food for degradation (and/or inhibition of aggregation) of Tau proteins and/or inhibition of phosphorylation of Tau proteins, the health functional food including, as an active ingredient, one or more selected from the group consisting of rifamycin, triflupromazine, and food acceptable salts thereof.

Another embodiment provides a health functional food for use in the prevention and/or amelioration of neurodegenerative brain disease, the health functional food including, as an active ingredient, rifapentine or food acceptable salts thereof.

Another non-claimed aspect provides a health functional food for the protection of cranial neuronal cells, the health functional food including, as an active ingredient, one or more selected from the group consisting of rifamycin, triflupromazine, and food acceptable salts thereof.

Another embodiment provides a health functional food for use in the prevention or amelioration of cognitive disorders or for the therapeutic use in improving memory, the health functional food including, as an active ingredient, rifapentine or food acceptable salts thereof.

SOLUTION TO PROBLEM

In the present specification, one or more selected from the group consisting of rifamycin, triflupromazine, and pharmaceutically acceptable salts thereof are confirmed to be therapeutically effective for neurodegenerative brain disease by identifying activities of inhibiting amyloid-beta aggregation, degrading amyloid-beta, degrading (and/or inhibition of aggregation of) Tau proteins, and/or inhibiting hyperphosphorylated Tau proteins, and thus, a use of these compounds is suggested for the inhibition and/or the degradation of amyloid-beta aggregation, and/or the degradation (and/or inhibition of aggregation) of Tau proteins and/or the inhibition of phosphorylation of Tau proteins, and/or the prevention and/or treatment of neurodegenerative brain disease. One or more selected from the group consisting of rifamycin, triflupromazine, and pharmaceutically acceptable salts thereof have excellent blood-brain barrier (BBB) permeability and thus effectively exert the activities of inhibiting and/or degrading amyloid-beta aggregation, and/or degrading

Tau proteins (and/or inhibition of Tau proteins aggregation), and/or inhibiting the phosphorylation of Tau proteins, in the brain, and/or preventing and/or treating neurodegenerative brain disease.

Accordingly, a non-claimed aspect provides a pharmaceutical composition for the inhibition and/or degradation of amyloid-beta aggregation, the pharmaceutical composition including, as an active ingredient, one or more selected from the group consisting of rifamycin, triflupromazine, and pharmaceutically acceptable salts thereof.

Another non-claimed aspect provides a pharmaceutical composition for degradation (and/or inhibition of aggregation) of Tau proteins and/or inhibition of phosphorylation of Tau proteins, the pharmaceutical composition including, as an active ingredient, one or more selected from the group consisting of rifamycin, triflupromazine, and pharmaceutically acceptable salts thereof.

Another non-claimed aspect provides a method of inhibiting and/or degrading amyloid-beta aggregation, the method including administering, to a subject in need of the inhibition and/or the degradation of amyloid-beta aggregation, a pharmaceutically effective amount of one or more selected from the group consisting of rifamycin, triflupromazine, and pharmaceutically acceptable salts thereof. The method may further include, before the administering, identifying a subject in need of amyloid-beta aggregation inhibition and/or degradation.

Another non-claimed aspect provides a method of degrading (and/or inhibiting of aggregation of) Tau proteins and/or inhibiting phosphorylation of Tau proteins, the method including administering, to a subject in need of degrading (and/or inhibiting of aggregation of) Tau proteins and/or inhibition of phosphorylation of Tau proteins, a pharmaceutically effective amount of one or more selected from the group consisting of rifamycin, triflupromazine, and pharmaceutically acceptable salts thereof. The method may further include, before the administering, identifying a subject in need of degradation (and/or inhibition of aggregation) of Tau proteins and/or inhibition of phosphorylation of Tau proteins.

An embodiment of the invention provides a pharmaceutical composition for use in the prevention and/or treatment of neurodegenerative brain disease, the pharmaceutical composition including, as an active ingredient, rifapentine or food acceptable salts thereof.

Another non-claimed aspect provides a method of preventing and/or treating neurodegenerative brain disease, the method including administering, to a subject in need of the prevention and/or treatment of neurodegenerative brain disease, a pharmaceutically effective amount of one or more selected from the group consisting of rifamycin, triflupromazine, and pharmaceutically acceptable salts thereof. The method may further include, before the administering, identifying a subject in need of the prevention and/or treatment of neurodegenerative brain disease.

Another embodiment provides a pharmaceutical composition for use in the prevention or amelioration of cognitive disorders or the therapeutic improvement of memory, the pharmaceutical composition including, as an active ingredient, rifapentine or pharmaceutically acceptable salts thereof.

Another non-claimed aspect provides a method of preventing or ameliorating cognitive disorders or improving memory, the method including administering, to a subject in need of the prevention or amelioration cognitive disorders or the improvement of memory, a pharmaceutically effective amount of one or more selected from the group consisting of rifamycin, triflupromazine, and pharmaceutically acceptable salts thereof. The method may further include, before the administering, identifying a subject in need of the prevention or amelioration of cognitive disorders or the improvement of memory.

Another non-claimed aspect provides a health functional food for amyloid-beta aggregation inhibition and/or degradation, the health functional food including, as an active ingredient, one or more selected from the group consisting of rifamycin, triflupromazine, and health functional food thereof.

Another non-claimed aspect provides a health functional food for degradation (and/or inhibition of aggregation) of Tau proteins and/or inhibition of phosphorylation of Tau proteins, the pharmaceutical composition including, as an active ingredient, one or more selected from the group consisting of rifamycin, triflupromazine, and food acceptable salts thereof.

Another embodiment provides a health functional food for use in the prevention and/or amelioration of neurodegenerative brain disease, the health functional food including, as an active ingredient, rifapentine or food acceptable salts thereof.

Another non-claimed aspect provides a health functional food for the protection of cranial neuronal cells, the health functional food including, as an active ingredient, one or more selected from the group consisting of rifamycin, triflupromazine, and food acceptable salts thereof.

Another embodiment provides a health functional food for use in the prevention or amelioration of cognitive disorders or the therapeutic improvement of memory, the health functional food including, as an active ingredient, rifapentine or food acceptable salts thereof.

Hereinafter, the present disclosure provided herein will be described in more detail.

The inventors of the present disclosure found that rifamycin or pharmaceutically acceptable salts thereof inhibits aggregation of amyloid-beta (see Example 1 and FIG. 1), and once the aggregation of amyloid-beta is induced, rifamycin or pharmaceutically acceptable salts thereof degrades aggregates thereof(see Examples 2 and 3, and FIGS. 3, 5, and 7).

Also, the effect of rifamycin according to the present disclosure on a decrease of the amyloid plaque and Tau

phosphorylation in an Alzheimer's animal model was identified and it was found that the number of amyloid plaques and the total area of the plaques were reduced (see Example 4, and FIGS. 8 to 10).

In addition, it was confirmed that rifamycin of the present disclosure improves cognitive functions and memory in an Alzheimer's animal model (see Example 6, FIGS. 13 and 14).

Also, the inventors of the present disclosure found that triflupromazine or pharmaceutically acceptable salts thereof inhibits aggregation of amyloid-beta (see Example 1 and FIG. 1), and once the aggregation of amyloid-beta is induced, rifamycin or pharmaceutically acceptable salts thereof degrades amyloid-beta aggregates thereof (see Example 2, and FIGS. 4 and 6).

Also, regarding the effect of triflupromazine according to the present disclosure on a decrease in the number of amyloid plaques and Tau phosphorylation in an Alzheimer's animal model, it was confirmed that triflupromazine reduced the number of amyloid plaques and the total area of plaques (see Example 4, and FIG. 11), degraded total Tau proteins, and inhibited phosphorylation of Tau proteins (see Example 5 and FIG. 12).

In the present specification, it was confirmed that since rifamycin or triflupromazine inhibits aggregation of amyloid-beta, degrades aggregates that have already been aggregated, degrades Tau protein, and inhibits phosphorylation of Tau, they have therapeutic effects on neurodegenerative brain disease on various diseases such as Alzheimer's and Parkinson's disease.

The term "rifamycin" used herein may refer to a group of many rifamycin derivatives. Rifamycin may be one selected from the group consisting of rifampicin, rifabutin, rifapentine, rifalazil, rifaximin, rifamdin, rifamycin B, rifamycin S, and rifamycin SV. The rifamycin according to the invention is rifapentine.

Rifapentine (according to the invention) may also be referred to as 3-[[(4-cyclopentyl-1-piperazinyl)imino]methyl]-rifamycin, and may be represented by Formula 1.

[Formula 1]

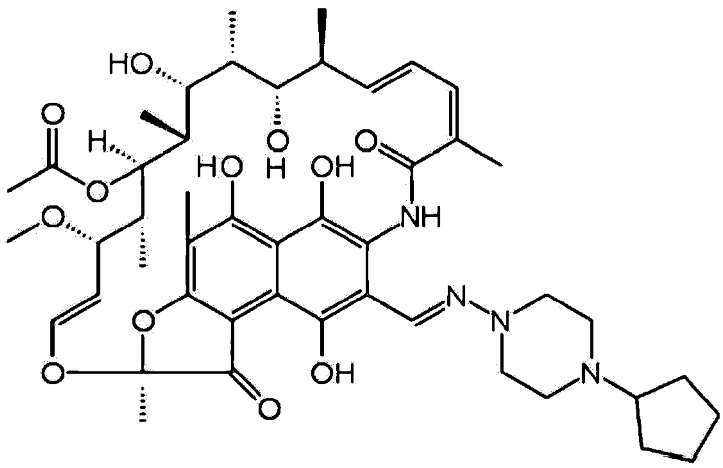

Rifampicin may be 5,6,9,17,19,21-hexahydroxy-23-methoxy-2,4,12,16,18,20,22-heptamethyl-8- [N- (4-methyl-1-piperazinyl) formimidoyl] -2,7- (epoxypentadeca [1,11,13] trienimino) -naphtho [2,1-b] furan-1,11 (2H) -dione 21-acetate, and may be represented by Formula 2.

[Formula 2]

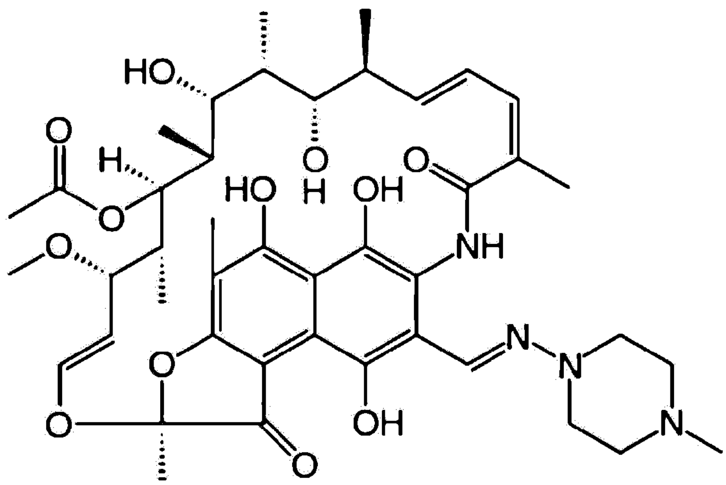

Rifabutin may be (S,12E,14S,15R,16S,17R,18R,19R,20S,21S,22E,24Z)-6,16,18,20-tetrahydroxy-1'-isobutyl-14-methoxy-7,9,15,17,19,21,25-heptamethyl-spiro[9,4-(epoxypentadeca[1,11,13]trienimino)-2H-furo[2",3":7,8]naphth[1,2-diimidazole-2,4"-piperidine]-5,10,26-(3H,9H)-trione-16-acetate, and may be represented by Formula 3.

[Formula 3]

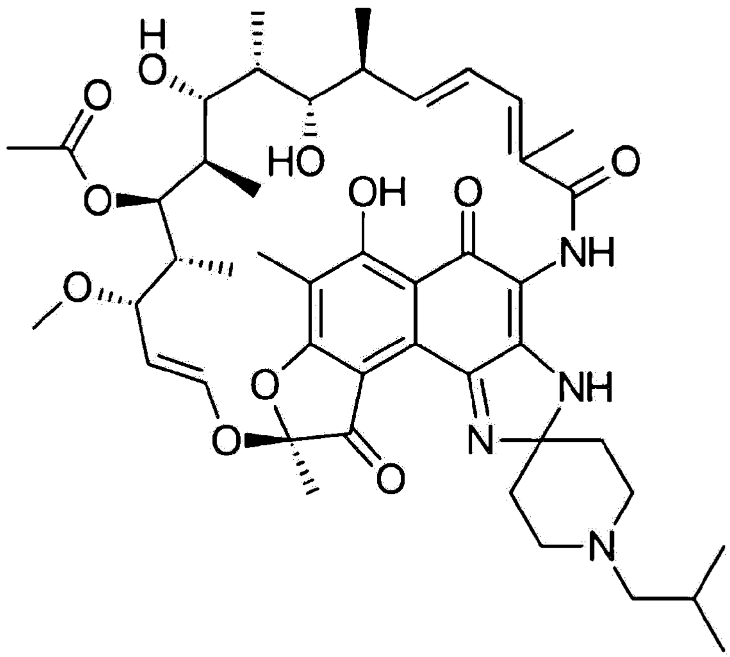

Rifalazil may be (2S,16Z,18E,20S,21S,22R,23R,24R,25S,26R,27S,28E)-5,12,21,23-tetrahydroxy-27-methoxy-2,4,16,20,22,24,26-heptamethyl-10-[4-(2-methylpropyl)piperazin-1-yl]-1,6,15-trioxo-1,2-dihydro-6H-2,7-(epoxypentadeca[1,11,13]trienoimino)[1]benzofuro[4,5-a]phenoxazin-25-yl acetate, and may be represented by Formula 4.

[Formula 4]

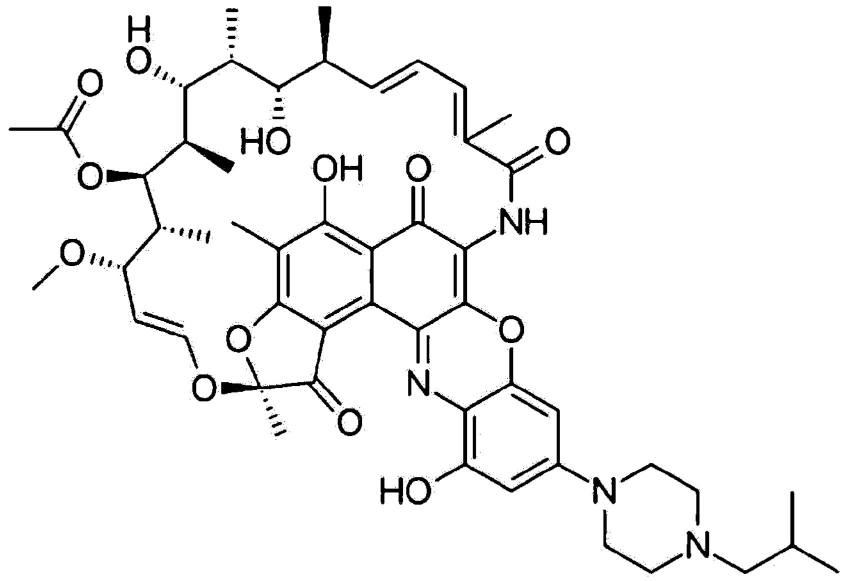

Rifaximin may be [2S- (2R *, 16Z, 18E, 20R *, 21R *, 22S *, 23S *, 24S *, 25R *, 26S *, 27R *, 22E)]-25-acetyloxy) -5,6 , 21,23-tetrahydroxy-27-methoxy-2,4,11,16,20,22,24,26-octamethyl-2,7- (epoxypentadeca [1,11,13] trienimino) benzofuro [4,5- e] pyrido [1,2-a] benzimidazole-1,15 (2H) -dione, and may be represented by the following Formula 5.

[Formula 5]

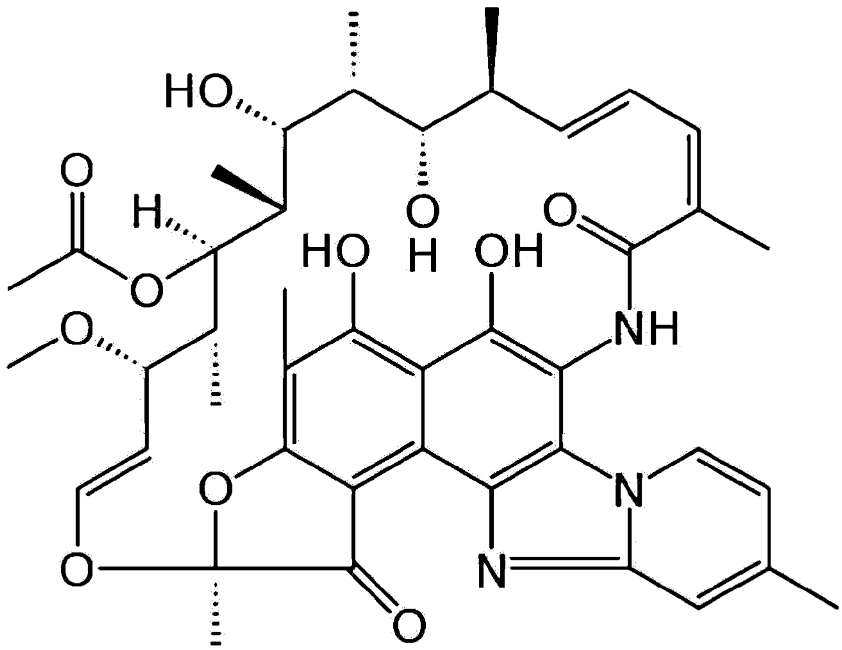

Rifamycin B may be {[(2S,12Z,14E,16S,17S,18R,19R,20R,21S,22R,23S,24E)-21-(acetyloxy)-5,6,17,19-tetrahydroxy-23-methoxy-2,4,12,16,18,20,22-heptamethyl-1,11-dioxo-1,2-dihydro-2,7-(epoxypentadeca[1,11,13]trienoimino)naphtho[2,1-b]furan-9-yl]oxy}acetic acid, and may be represented byFormula 6.

[Formula 6]

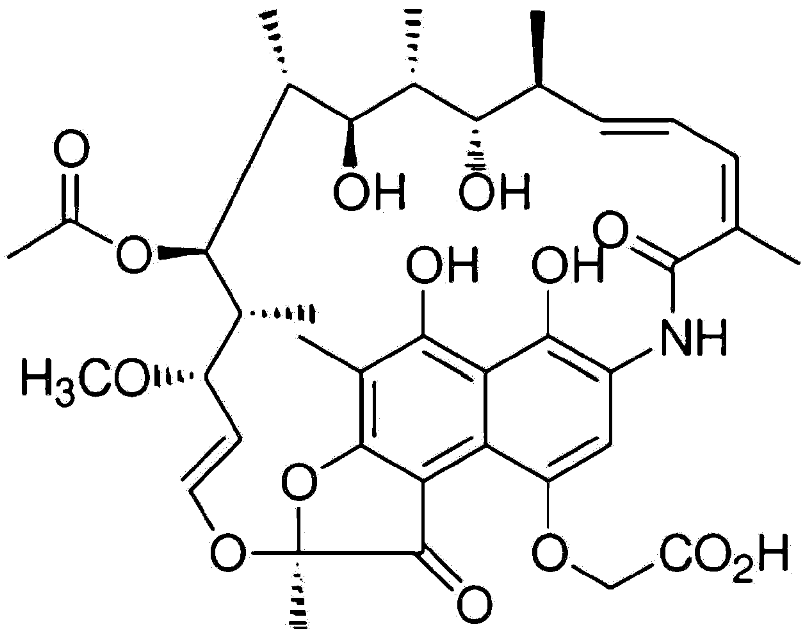

Rifamycin S may be 5,17,19,21-tetrahydroxy-23-methoxy-2,4,12,16,18,20,22-heptamethyl-2,7-(epoxypentadeca[1,11,13]trienimino)naphtho[2,1-b]furan-1,6,9,11 (2H)-tetrone 21-acetate, and may be represented by Formula 7.

[Formula 7]

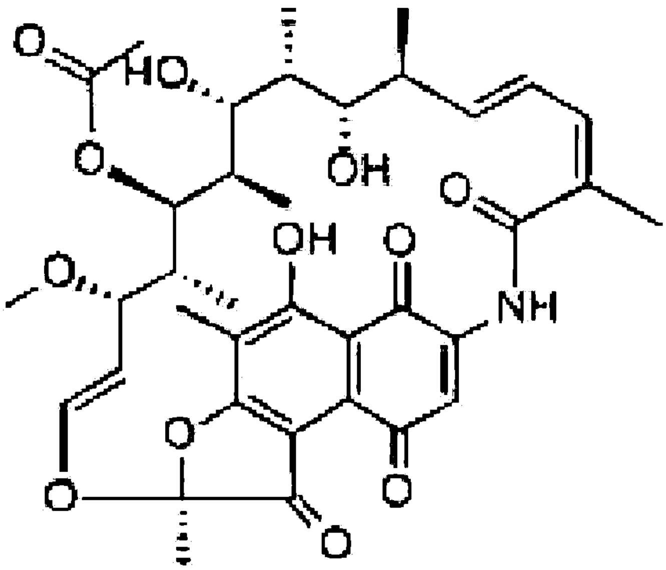

Rifamycin SV may be 5,6,9,17, 19,21-Hexahydroxy-23-methoxy-2,4,12,16,18,20,22-heptamethyl-2,7-(epoxypentadeca[1,11,13]trienimino)naphtho[2,1-b]furan-1,11(2H)-dione 21-acetate, and may be represented by Formula 8.

[Formula 8]

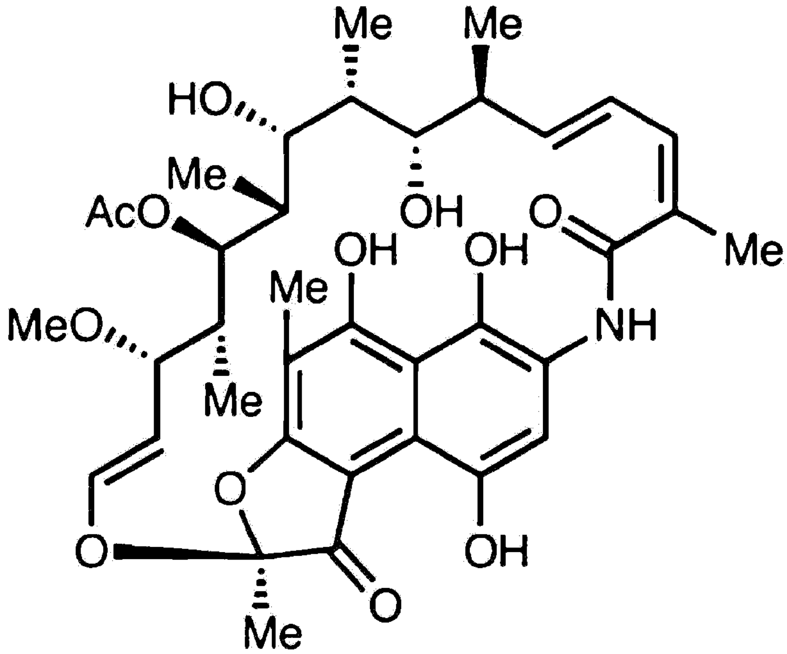

Rifamdin may be 3-[[[4-(2-Methylpropyl)-1-piperazinyl]imino]methyl]rifamycin, and may be represented by Formula 9.

[Formula 9]

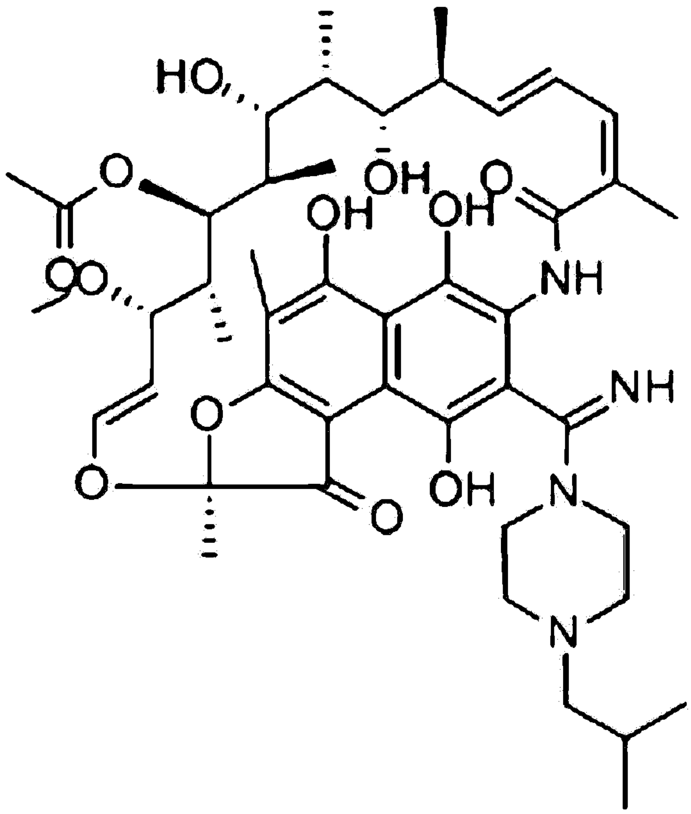

Triflupromazine (dimethyl ({3-[2-(trifluoromethyl)-1 OH-phenothiazin-1 0-yl]propyl})amine; CAS No. 146-54-3; see Formula 10) is mainly used in the treatment of mental and emotional disorders, such as schizophrenia or the like. However, the effect of triflupromazine on the inhibition of amyloid-beta aggregation and/or degradation, and/or degradation (and/or inhibition of aggregation) of Tau proteins, and/or inhibition of phosphorylation of Tau proteins, and/or prevention and/or treatment of neurodegenerative brain disease has not been known.

[Formula 10]

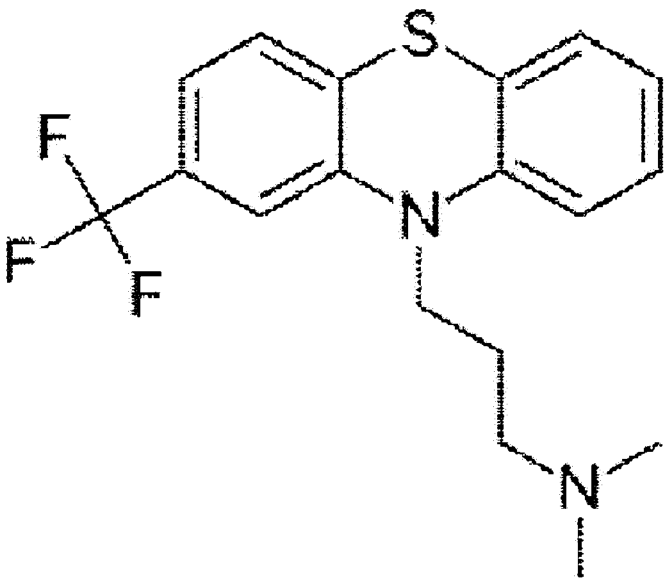

Human amyloid-beta is a peptide molecule containing about 36-43 amino acids, and is a major component of amyloid plaques expressed in the brain of Alzheimer's patients, and is known to be involved in the development of Alzheimer's disease. The amyloid-beta peptide molecule may be obtained by cleaving an amyloid precursor protein (APP; UniProtKB P05067) with beta secretase and gamma secretase. Amyloid-beta peptide molecules aggregate to form neuronal toxic oligomers, leading to neurodegenerative brain disease.

The Tau protein consists of four parts including the N-terminal overhang, the proline aggregation domain, the microorganism binding domain and the C-terminal (Mandelkow et al., Acta.Neuropathol., 103, 26-35, 1996), and abnormal hyperphosphorylation or deformation of Tau in neurons of the central nervous system is known to cause neurodegenerative brain diseases such as Parkinson's disease and Tauopathy.

The term 'neurodegenerative brain disease' used herein refers to any disease that is associated with degenerative changes in the brain, in particular, all diseases (brain diseases) that can be caused by one or more factors selected from the aggregation of amyloid-beta, the aggregation of Tau proteins, and hyperphosphorylation of Tau proteins, in the brain and/or cranial neuronal cells. In one embodiment, the neurodegenerative brain disease may be, for example, Alzheimer's disease, Parkinson's disease, Huntington's disease, mild cognitive impairment, cerebral amyloid angiopathy, down syndrome, amyloid stroke, systemic amyloid disease, Dutch-type amyloidosis, Nieman-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, or frontotemporal dementia. However, embodiments are not limited thereto, and the neurodegenerative brain disease may be any disease that is caused by the aggregation of amyloid-beta, the aggregation of Tau proteins, and/or the phosphorylation of Tau proteins.

The term 'treatment' used herein refers to alleviation or amelioration of a pathological condition, reduction of a site of a disease, delay or amelioration of disease progression, amelioration, alleviation, or stabilization of a disease state or symptom, partial or complete recovery, prolongation of survival, other beneficial treatment outcomes, etc. The term 'prevention' used herein includes all mechanisms and/or effects that act on a subject that does not have a particular disease to prevent the disease from developing, to delay the onset of the disease, or to reduce the frequency of the disease. The term 'protection of cranial neuronal cells' used herein includes all mechanisms and/or effects that inhibit the damage and/or death of cranial neuronal cells.

The pharmaceutically or food acceptable salts may be any salt that is commonly used in the pharmaceutical field. In one embodiment, the pharmaceutically acceptable salts may be one or more selected from salts formed using: non-toxic inorganic acids such as hydrochloric acid, bromic acid, sulfonic acid, amido sulfuric acid, phosphoric acid,

nitric acid, and the like; or organic acids such as acetic acid, propionic acid, succinic acid, glycolic acid, stearic acid, lactic acid, tartaric acid, citric acid, paratoluenesulfonic acid, methanesulfonic acid, and the like, and, for example, may be hydrochloride. However, embodiments are not limited thereto.

The pharmaceutical composition provided herein may be used for

(1) subjects (patients) who have a higher level or a higher risk of aggregation of amyloid-beta than normal individuals;
(2) subjects (patients) who have a higher level or a higher risk of aggregation of Tau proteins than normal individuals;
(3) subjects (patients) who have a higher level or a higher risk of phosphorylation of Tau proteins than normal individuals; and
(4) a subject (patient) selected from the group consisting of subjects (patients) corresponding to one or more of (1) to (3).

The aggregation level of the amyloid-beta or Tau proteins may refer to the amount (concentration) of the amyloid-beta aggregates or the Tau protein aggregates or the ratio of the amyloid-beta aggregates or the Tau protein aggregates to the total amyloid-beta or the total Tau protein.

The phosphorylation level of the Tau protein may refer to the amount (concentration) of phosphorylated Tau protein or the ratio of phosphorylated Tau protein to total Tau protein.

The "normal' may refer to a subject that does not have "neurodegenerative brain disease" defined above or a brain tissue or brain cells (cranial neuronal cells) which are separated from and/or cultured from the subject, from among subjects which are homogeneous with the subject (patient) to which the pharmaceutical composition is applied.

In one embodiment, the pharmaceutical composition may further include, in addition to the active ingredient (rifapentine or a pharmaceutically acceptable salt thereof), one or more additives selected from the group consisting of a carrier, an excipient, a diluent, a filler, an extender, a wetting agent, a disintegrant, an emulsifier (a surfactant), a lubricant, a sweetener, a flavor, a suspension, a preservative and the like, all of which are pharmaceutically acceptable. The additives may be appropriately adjusted depending on the dosage form to which the pharmaceutical composition is applied, and may include one or more selected from all additives which can be used conventionally in the pharmaceutical field. In one embodiment, the pharmaceutically acceptable carrier may be one or more selected from the group consisting of lactose, dextrose, sucrose, trehalose, arginine, histidine, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, etc, all of which are conventionally used for formulation of drugs. However, embodiments are not limited thereto.

An effective amount of the active ingredient (rifapentine or a pharmaceutically acceptable salt thereof), or the pharmaceutical composition may be administered orally or parenterally. In the case of parenteral administration, the administering may be performed by intravenous injection, subcutaneous injection, intramuscular injection, intraperitoneal injection, endothelial administration, intranasal administration, pulmonary administration, rectal administration, or local administration to lesion sites. In the case of the oral administration, the pharmaceutical composition may be prepared in such a way that the active ingredient is coated not to be degraded in the stomach, or may be prepared in a formulation that is protectable from degradation in the stomach.

In addition, the term 'active ingredient' used herein refers to a bioactive substance described herein (for example, rifamycin, triflupromazine, or pharmaceutically acceptable salts thereof) to achieve pharmaceutical activities described in the present specification (for example, the treatment of neurodegenerative brain disease). This is distinguishable from the administering of rifamycin or triflupromazine alone or in combination thereof with other substances, or the additional administering thereof for the purpose of the known activities of rifamycin or triflupromazine (for example, antibiotic activity, schizophrenia treatment) for the treatment of the diseases referred to herein. That is, rifapentine may be administered as the sole active ingredient for direct therapeutic effects of neurodegenerative brain disease.

As described herein, the term "pharmaceutically effective amount" may refer to the amount or dosage of an active ingredient (rifapentine or pharmaceutically acceptable salts etc.) that can exhibit the desired pharmacological effect, in the pharmaceutical composition. The effective amount or active ingredient may be appropriately determined by factors, such as a formulation method, mode of administration, age, weight, gender, pathological condition, food, time of administration, intervals of administration, route of administration, rate of excretion and sensitivity of reaction. For example, the daily or single dose of the active ingredient may be 0.0001 mg/kg to 1000 mg/kg (body weight), 0.001 to 500 mg/kg, 0.01 to 100 mg/kg, 0.1 to 50 mg/kg, or 0.5 to 20 mg/kg, but is not limited thereto. The daily or single dose may be prepared into a single formulation in unit dosage form, formulated in appropriate quantities, or incorporated into a multi-dose container.

The amount of the active ingredient (that is, rifapentine or pharmaceutically acceptable salts thereof) in the pharmaceutical composition may be appropriately adjusted according to the use form of the pharmaceutical composition, the condition of the patient, the target effect, and the like, and may be , for example, 0.0001 wt% to 99.9 wt%, 0.001 wt% to 99.9 wt%, 0.01 wt% to 99.9 wt%, 0.1 wt% to 99.9 wt%, 0.5 wt% to 99.9 wt%, 1 wt% to 99.9 wt%, 5 wt% to 99.9

wt%, 10 wt% to 99.9 wt%, 15 wt% to 99.9 wt%, 20 wt% to 99.9 wt%, 25 wt% to 99.9 wt%, 30 wt% to 99.9 wt%, 35 wt% to 99.9 wt%, 40 wt% to 99.9 wt%, 45 wt% to 99.9 wt%, 50 wt% to 99.9 wt%, 55 wt% to 99.9 wt%, 0.0001 wt% to 90 wt%, 0.001 wt% to 90 wt%, 0.01 wt% to 90 wt%, 0.1 wt% to 90 wt%, 0.5 wt% to 90 wt%, 1 wt% to 90 wt%, 5 wt% to 90 wt%, 10 wt% to 90 wt%, 15 wt% to 90 wt%, 20 wt% to 90 wt%, 25 wt% to 90 wt%, 30 wt% to 90 wt%, 35 wt% to 90 wt%, 40 wt% to 90 wt%, 45 wt% to 90 wt%, 50 wt% to 90 wt%, 55 wt% to 90 wt%, 0.0001 wt% to 70 wt%, 0.001 wt% to 70 wt%, 0.01 wt% to 70 wt%, 0.1 wt% to 70 wt%, 0.5 wt% to 70 wt%, 1 wt% to 70 wt%, 5 wt% to 70 wt%, 10 wt% to 70 wt%, 15 wt% to 70 wt%, 20 wt% to 70 wt%, 25 wt% to 70 wt%, 30 wt% to 70 wt%, 35 wt% to 70 wt%, 40 wt% to 70 wt%, 45 wt% to 70 wt%, 50 wt% to 70 wt%, 55 wt% to 70 wt%, 0.0001 wt% to 50 wt%, 0.001 wt% to 50 wt%, 0.01 wt% to 50 wt%, 0.1 wt% to 50 wt%, 0.5 wt% to 50 wt%, 1 wt% to 50 wt%, 5 wt% to 50 wt%, 10 wt% to 50 wt%, 15 wt% to 50 wt%, 20 wt% to 50 wt%, 25 wt% to 50 wt%, 30 wt% to 50 wt%, 35 wt% to 50 wt%, 40 wt% to 50 wt%, 45 wt% to 50 wt%, 0.0001 wt% to 40 wt%, 0.001 wt% to 40 wt%, 0.01 wt% to 40 wt%, 0.1 wt% to 40 wt%, 0.5 wt% to 40 wt%, 1 wt% to 40 wt%, 5 wt% to 40 wt%, 10 wt% to 40 wt%, 15 wt% to 40 wt%, 20 wt% to 40 wt%, 25 wt% to 40 wt%, 30 wt% to 40 wt%, or 35 wt% to 40 wt%, but is not limited thereto.

The pharmaceutical composition may be in the form of solutions, suspensions, syrups or emulsions in aqueous or oily media, or may be formulated in the form of powder, granules, tablets or capsules, etc, and for formulation, the pharmaceutical composition may further include a dispersant or a stabilizer.

Subjects to be administered with the pharmaceutical composition may be primates including humans, monkeys, etc., rodents including mice, rats, etc., mammals including dogs, cats, cattle, pigs, sheep, goats, horses, etc., or cells and tissues isolated from the mammals, or cultures thereof.

The health functional food is a food prepared by using nutrients or ingredients that are useful for the human body (hereinafter referred to as 'functional ingredients'), which are easily deficient in daily meals, and maintain health or prevent and/or ameliorate certain diseases or symptoms, and the health functional food has no specific restrictions on the final product form thereof. For example, the health functional food may be selected from the group consisting of various foods, beverage compositions, food additives, and the like, but is not limited thereto.

The amount of the active ingredient (that is, rifapentine or pharmaceutically acceptable salts thereof) contained in the health functional food may be appropriately adjusted depending on the form of the food, desired use, etc. and is not particularly limited, and may be, for example, based on the total weight of food, in the range of 0.0001 wt% to 99 wt%, 0.0001 wt% to 95 wt%, 0.0001 wt% to 90 wt%, 0.0001 wt% to 80 wt%, 0.0001 wt% to 50 wt%, 0.001 wt% to 99 wt%, 0.001 wt% to 95 wt%, 0.001 wt% to 90 wt%, 0.001 wt% to 80 wt%, 0.001 wt% to 50 wt%, 0.01 wt% to 99 wt%, 0.01 wt% to 95 wt%, 0.01 wt% to 90 wt%, 0.01 wt% to 80 wt%, 0.01 wt% to 50 wt%, 0.1 wt% to 99 wt%, 0.1 wt% to 95 wt%, 0.1 wt% to 90 wt%, 0.1 wt% to 80 wt%, 0.1 wt% to 50 wt%, 0.1 wt% to 30 wt%, 0.1 wt% to 10 wt%, 1 wt% to 99 wt%, 1 wt% to 95 wt%, 1 wt% to 90 wt%, 1 wt% to 80 wt%, 1 wt% to 50 wt%, 1 wt% to 30 wt%, 1 wt% to 10 wt%, 10 wt% to 99 wt%, 10 wt% to 95 wt%, 10 wt% to 90 wt%, 10 wt% to 80 wt%, 10 wt% to 50 wt%, 10 wt% to 30 wt%, 25 wt% to 99 wt%, 25 wt% to 95 wt%, 25 wt% to 90 wt%, 25 wt% to 80 wt%, 25 wt% to 50 wt%, 25 wt% to 30 wt%, 40 wt% to 99 wt%, 40 wt% to 95 wt%, 40 wt% to 90 wt%, 40 wt% to 80 wt%, 40 wt% to 50 wt%, 50 wt% to 99 wt%, 50 wt% to 95 wt%, 50 wt% to 90 wt%, 50 wt% to 80 wt%, 60 wt% to 99 wt%, 60 wt% to 95 wt%, 60 wt% to 90 wt%, or 60 wt% to 80 wt%, but is not limited thereto.

The health functional food may include one or more selected from the group consisting of various nutrients, vitamins, minerals (electrolytes), flavors such as synthetic flavors or natural flavors, colorants, enhancers (cheese, chocolate, etc.), pectic acid or salts thereof, alginic acid or salts thereof, organic acids, protective colloidal thickeners, pH adjusters, stabilizers, preservatives, glycerin, alcohols, carbonation agents used in carbonated drinks, and the like. The ratio of such additives is generally selected from 0.001 to about 20 parts by weight based on 100 parts by weight of the total health functional food, but is not limited thereto.

## ADVANTAGEOUS EFFECTS OF DISCLOSURE

According to the technology provided herein, due to the use of rifamycin, triflupromazine, or a pharmaceutically acceptable salt thereof as an active ingredient, the excellent inhibition and/or degradation effect of amyloid-beta aggregation, the degradation (and/or inhibition of aggregation) of Tau proteins, and inhibition of hyperphosphorylation of Tau proteins can be obtained, and excellent blood brain barrier permeability can be obtained and thus the active ingredient can be successfully applied to the brain tissue. Thus, the technology can be usefully applied for the prevention and/or treatment of various neurodegenerative brain diseases related to amyloid-beta aggregation, Tau protein aggregation, and/or hyperphosphorylated Tau proteins.

## BRIEF DESCRIPTION OF DRAWINGS

FIG. 1 shows the graph of results showing that amyloid-beta (Aβ) aggregation inhibition was identified by Thioflavin T analysis, and in particular, the amyloid-beta aggregation inhibition effect of rifapentine (control: a group treated with $Aβ_{1-42}$ 50 μM; 2: a group treated with $Aβ_{1-42}$ 50μM + rifapentine 2 μM).

FIG. 2 shows the graph of results showing that inhibition of Aβ aggregation was identified by Thioflavin T analysis, and in partiulcar, the Aβ aggregation inhibition effect of triflupromazine hydrochloride (control: a group treated with $Aβ_{1-42}$50 μM; 2: a group treated with $Aβ_{1-42}$50 μM + triflupromazine hydrochloride 2 μM).

FIG. 3 shows the graph of results showing that Aβ disaggregation was identified by Thioflavin T analysis, and in particular, the Aβ disaggregation effect of rifapentine (control: a group treated with $Aβ_{1-42}$ 25 μM; 50: a group treated with $Aβ_{1-42}$ 25 μM + rifapentine 50 μM; 500: a group treated with $Aβ_{1-42}$ 25 μM + rifapentine 500 μM).

FIG. 4 shows the graph of results showing that Aβ disaggregation was identified by Thioflavin T analysis, and in particular, the Aβ disaggregation effect of triflupromazine hydrochloride (control: a group treated with $A_{β1-42}$ 25 μM; 50: a group treated with $Aβ_{1-42}$ 25 μM +triflupromazine hydrochloride 50 μM; 500: a group treated with $Aβ_{1-42}$ 25 μM + triflupromazine hydrochloride 500 μM).

FIG. 5 shows an electrophoretic image by which Aβ disaggregation was confirmed by PICUP-based SDS-PAGE, showing the Aβ disaggregation effect of rifapentine.

FIG. 6 shows an electrophoretic image by which Aβ disaggregation was confirmed by PICUP-based SDS-PAGE, showing the effect of Aβ disaggregation of triflupromazine hydrochloride.

FIG. 7 shows the graph of results showing that Aβ disaggregation was confirmed by Thioflavin T analysis, and in particular, the Aβ disaggregation effect of rifamycin-based derivatives including rifapentine (rifampicin, rifabutin, rifamycin SV, rifamdin, rifamycin S, rifaximin, and rifamycin B) (control: groups treated with $Aβ_{1-42}$ 50 μM were aggregated for 3 days or 5 days for each; Treatment groups: $Aβ_{1-42}$ 50 μM was aggregated for 3 days + treatment with 50 μM rifamycin-based derivatives followed by two days of reaction)

FIG. 8 shows the image of stained tissue by which the degradation of Aβ plaques in transgenic Alzheimer's dementia animal (APP/PS1 TG) was confirmed by using 6E10 antibody, showing the Aβ aggregation effect of rifapentine.

FIG. 9 shows the graph of results in which the degradation of Aβ plaques in transgenic Alzheimer's dementia animals (APP/PS1 TG) was confirmed by using 6E10 antibody, showing the Aβ disaggregation effect of rifapentine; the total number of Aβ plaques in the cerebral hemisphere, the number of Aβ plaques in the hippocampus of the cerebral hemisphere, and the number of Aβ plaques in the cerebral cortex of the cerebral hemispheres, in the rifapentine treated group, were significantly decreased compared with those of the TG mice administered with a vehicle.

FIG. 10 shows the graph of results in which the degradation of Aβ plaques in transgenic Alzheimer's dementia animals (APP/PS1 TG) was confirmed by using 6E10 antibody, showing the Aβ disaggregation effect of rifapentine; the total area of Aβ plaques in the cerebral hemisphere, the total area of Aβ plaques in the hippocampus of the cerebral hemisphere, and the total area of Aβ plaques in the cerebral cortex of the cerebral hemispheres, in the rifapentine treated group, were significantly decreased compared with those of the TG mice administered with a vehicle.

FIG. 11 shows the image of stained tissue by which the degradation of Aβ plaque in transgenic Alzheimer's animal model (5X FAD) was confirmed by using Thioflavin S analysis, showing the Aβ disaggregation effect of triflupromazine hydrochloride.

FIG. 12 shows an electrophoresis image of the transgenic Alzheimer animal model (5X FAD) in which phosphorylated Tau proteins were confirmed by SDS-PAGE, showing the Tau proteins phosphorylation inhibition effect of triflupromazine hydrochloride.

FIG. 13 shows the results of the Y-maze test (Y-shaped maze evaluation) 8 weeks after the transgenic Alzheimer's dementia animals (APP/PS1 TG) were administered with rifapentine, and the results are values of the spontaneous alteration (%) which refers to the relative frequency at which the experimental animal enters the maze sequentially, showing that the memory level improves closer to the positive control group, WT-Veh, compared to the negative control group, TG-Veh.

FIG. 14 shows the results of the novel object recognition test (novel material recognition test) 8 weeks after the transgenic Alzheimer's dementia animals (APP/PS1 TG) were administered with rifapentine, and the results are the preference index which refers to the relative frequency at which the experimental animal searches for a novel object, showing that memory is increased closer to the positive control group, WT-Veh, compared to the negative control group, TG-Veh.

## MODE OF DISCLOSURE

Hereinafter, the present disclosure will be described in more detail with reference to Examples, which are merely illustrative and are not intended to limit the scope of the present disclosure.

The examples relating to rifapentine are part of the claimed invention; the other examples are for reference.

**Example 1: Aβ aggregation inhibition test**

1.1 Preparation of Sample

Rifapentine was dissolved to a concentration of 5 mM in dimethylsulfoxide (DMSO). Then, the compound was used diluted by using 6% DMSO (in DW) and used.

Triflupromazine hydrochloride (triflupromazine used in all the examples below is triflupromazine hydrochloride) was dissolved to a concentration of 5 mM in DMSO. The compound was then used diluted with 6% DMSO (in DW).

The Aβ solution was prepared by dissolving human Aβ1-42 monomers (UniProtKB-P05067, a.a.672 - 713) to 10mM concentration in DMSO. Thereafter, the mixture was diluted to 100 uM by using DW and stored on ice.

On the other hand, Thioflavin-T (Sigma-Aldrich) was dissolved to the concentration of 5 mM in 50 mM glycine buffer (pH 8.5). Then, the resultant solution was diluted to the concentration of 50 μM by using 50 mM glycine buffer (pH 8.5) and stored in the dark while light was blocked.

1.2 Measurement of Aβ aggregation inhibition effect

The Aβ solution prepared above was placed at the concentration of 50 μM in 1.5 mL microcentrifuge tubes, each of which was treated with 2 μM rifapentine or triflupromazine, followed by reacting at 37 °C for 72 hours.

25 μL of the reaction solution in which the reaction was completed was added to each well of a 96-well fluorescence assay plate, and 75 μL of the Thioflavin-T solution prepared above was added to each well. After reacting for 5 minutes in the dark at room temperature, fluorescence values were measured at excitation 450 nm and emission 485 nm by using a multi-mode microplate reader.

The fluorescence value of the group (control) treated with Aβ alone and aggregated for 72 hours was considered as 100%, and the measured fluorescence values were expressed as a relative value thereto, and results thereof are shown in FIG. 1 and FIG. 2.

FIG. 1 shows the Aβ aggregation inhibition effect of rifapentine (control: a group treated with $A\beta_{1-42}$ 50 μM; 2: a group treated with $A\beta_{1-42}$ 50 μM + rifapentine 2 μM), and when treated with rifapentine (2 μM), the Aβ aggregation degree was about 40% or less compared to the control. Accordingly, it can be seen that rifapentine showed about 60% or more of amyloid-beta aggregation inhibition effect compared to the control.

FIG. 2 shows the Aβ aggregation inhibition effect of triflupromazine (control: a group treated with $A\beta_{1-42}$ 50 μM; 2: a group treated with $A\beta_{1-42}$ 50 μM + triflupromazine 2 μM), and when treated with triflupromazine (2 μM), the Aβ aggregation degree was about 70% or less compared to the control. Accordingly, it can be seen that triflupromazine showed about 30% or more of amyloid-beta aggregation inhibition effect compared to the control.

In addition, by performing the same test as described above for 20 μM of various compounds using 50 μM Aβ, the degree of Aβ aggregation was measured, and the result is expressed as a relative value for control (100%). Table 1 shows:

Table 1

| NO. | Treatment compound | Aggregation (%) |
|---|---|---|
| 1 | Aβ(0 day) | 0 |
| 2 | Aβ (3 days) (control) | 100.00 |
| 3 | Aβ + Rifapentine (3 days) | 13.35 |
| 4 | Aβ + Triflupromazine HCl (3 days) | 49.84 |
| 5 | Aβ + Thiothixene (3 days) | 125.23 |
| 6 | Aβ + Risperidone (3 days) | 117.68 |
| 7 | Aβ + Clozapine (3 day) | 99.14 |
| 8 | Aβ + Amisulpride (3 day) | 96.29 |
| 9 | Aβ + Mementine (3 days) | 95.84 |

As shown in Table 1, it can be seen that the Aβ aggregation inhibition activity of rifapentine and triflupromazine is significantly higher than other compounds.

**Example 2: Aβ disaggregation test**

2.1. Sample Preparation

Rifapentine was dissolved to a concentration of 5 mM in dimethylsulfoxide (DMSO). The compound was then used diluted with 6% DMSO (in DW).

Triflupromazine hydrochloride was dissolved to the concentration of 5 mM in DMSO. The compound was then used diluted with 6% DMSO (in DW).

The Aβ solution was prepared by dissolving Aβ 1-42 monomers to the concentration of 10 mM in DMSO. Thereafter, the mixture was diluted to 100 μM by using DW and stored on ice.

On the other hand, Thioflavin-T was dissolved to the concentration of 5 mM in 50 mM glycine buffer (pH 8.5). Then, the resultant solution was diluted to the concentration of 50 μM by using 50 mM glycine buffer (pH 8.5) and stored in the dark while light was blocked.

**2.2. Aβ disaggregation test by Thioflavin T analysis**

The Aβ solution prepared in Example 2.1. was placed in 1.5 mL microcentrifuge tubes, each having 25 μM of the Aβ solution, and then reacted at 37 °C for 72 hours.

Each 1.5 mL microcentrifuge tube was treated with rifapentine and triflupromazine at different concentrations of 50 μM or 500 μM, followed by reaction at 37 °C for 72 hours.

25 μL of the reaction solution in which the reaction was completed was added to each well of a 96-well fluorescence assay plate, and 75 μL of the Thioflavin-T solution prepared above was added to each well. After reacting for 5 minutes in the dark at room temperature, fluorescence values were measured at excitation 450 nm and emission 485 nm by using a multi-mode microplate reader.

The fluorescence value of the group (control) treated with Aβ alone and aggregated for 72 hours was considered as 100%, and the measured fluorescence values were expressed as a relative value thereto, and results thereof are shown in FIG. 3 and FIG. 4.

FIG. 3 shows the Aβ disaggregation effect of rifapentine (control: a group treated with $A\beta_{1-42}$ 25 μM; 50: a group treated with $A\beta_{1-42}$ 25 μM + rifapentine 50 μM; and 500: a group treated with $A\beta_{1-42}$ 25 μM + 500 μM rifapentine), and when treated with rifapentine, the degree of Aβ aggregation was about 10% or less (when treated with 50 μM rifapentine ) or about 1% or less (when treated with 500 μM rifapentine) compared to the control. Accordingly, it can be seen that rifapentine shows the amyloid-beta disaggregation effect of about 90% or more (when treated with 50 μM rifapentine) or about 99% or more (when treated with 500 μM treatment). This means that rifapentine has a concentration-dependent disaggregation effect.

FIG. 4 shows the Aβ disaggregation effect of triflupromazine (control: a group treated with $A\beta_{1-42}$ 25 μM; 50: a group treated with $A\beta_{1-42}$ 25 μM + 50 μM triflupromazine; and 500: a group treated with $A\beta_{1-42}$ 25 μM + 500 μM triflupromazine), and when treated with triflupromazine, the degree of Aβ aggregation was, compared to the control, about 65% or less (when treated with 50 μM triflupromazine) or about 25% or less (when treated with 500 μM triflupromazine). Accordingly, it can be seen that triflupromazine shows the amyloid-beta disaggregation effect of 35% or more (when treated with 50 μM triflupromazine) or about 75% or more (when treated with 500 μM triflupromazine), compared to the control. This means that triflupromazine has a concentration-dependent disaggregation effect.

**2.3. Aβ disaggregation test by SDS-PAGE analysis**

In order to confirm Aβ disaggregation in the sample prepared in Example 2.1, SDS-PAGE analysis was performed as follows.

In detail, the stock of ammonium persulfate (APS; 200 mM)and tris (2,2'-bipyridyl)dichlororuthenium (II) hexahydrate (Ru; 10 mM) was prepared in an amber tube (Axygen, MCT-150-X) by using a photo-induced cross-linking of unmodified proteins (PICUP) buffer (0.1 M sodium phosphate (pH 7.4)). The stock was diluted 10-fold in PICUP buffer (APS: 20 mM / Ru: 1 mM). 10 μL of each sample prepared in Example 2.1 was dispensed at the bottom of a 0.5 mL tube (Axygen, PCR-05-C). 1 μL of APS (20 mM) was dispensed on the tube wall and 1 μL of Ru (1 mM) on the tube cap. At this time, in order to prevent the reaction from proceeding, the sample was not mixed with APS and Ru. After closing the tube cap and spin down using a centrifuge, the tube was removed from the centrifuge and exposed to light 3 times per second using an incandescent lamp. 3 μL of 5X reducing sample buffer (w/ 5% β-mercaptoethanol) was added to each tube. Samples were heated at 95-100 °C. for 5 minutes and then cooled on ice.

Then, the PICUP-treated samples were loaded on 4-20% (w/v) tris/glycine gel and electrophoresed at 100V for 1 hour and 30 minutes. After electrophoresis, the gel was separated and confirmed by silver staining.

The obtained electrophoresis results are shown in FIGS. 5 and 6. Except for the left-most size marker lane in

FIGS. 5 and 6, the first lane (Aβ aggregates (-) + compound (-)) shows the result of the $Aβ_{42}$ only 0 day sample (day 0 after the treatment with 25 μM Aβ, no amyloid-beta aggregation occurred), the second lane (Aβaggregates (+) + compound (-)) shows the result of the $Aβ_{42}$ only 3 + 3 day sample (treatment with 25μM Aβ and incubation for 3 days to induce amyloid-beta aggregation, and incubation for 3 days), and the third lane (Aβaggregates (+) + 50μM compound) and the fourth lane (Aβaggregates (+) + 500μM compound) show the Aβ disaggregation effect of the $Aβ_{42}$ + compounds (50μM or 500μM) 3 + 3 day samples (treatment with 25μM Aβ, incubation for 3 days to induce amyloid-beta aggregation, treatment with rifapentine or triflupromazine at a concentration of 50 μM or 500 μM and further incubation for 3 days). As shown in FIG. 5 and FIG. 6, it can be seen that as the concentration of rifapentine or triflupromazine is increased, bands of aggregated Aβ fibrils and dimers (about 10 kDa) are blurred. These results show that rifapentine or triflupromazine has a concentration-dependent Aβ disaggregation activity.

**Example 3: Aβ disaggregation test of rifamycin-based derivatives**

**3.1. Sample Preparation**

Compounds (rifapentine, rifampicin, rifabutin, rifamycin SV, rifamycin B, rifamycin S, rifaximin, rifamdine; above-described rifamycin derivatives) were dissolved to the concentration of 5 mM in DMSO. The compound was then used diluted with 6% DMSO (in DW).

The Aβ solution was prepared by dissolving Aβ 1-42 monomers to the concentration of 10 mM in DMSO. Thereafter, the mixture was diluted to 100 μM by using DW and stored on ice.

On the other hand, Thioflavin-T was dissolved to the concentration of 5 mM in 50 mM glycine buffer (pH 8.5). Then, the resultant solution was diluted to the concentration of 50 μM by using 50 mM glycine buffer (pH 8.5) and stored in the dark while light was blocked.

**3.2. Aβ disaggregation test by Thioflavin T analysis**

The Aβ solution prepared in Example 3.1. was placed in 1.5 mL microcentrifuge tubes, each having 50 μM of the Aβ solution, and then reacted at 37 °C for 72 hours.

Each 1.5 mL microcentrifuge tube was treated with 50 μM rifapentine-based derivatives, followed by reaction at 37 °C for 48 hours.

25 μL of the reaction solution in which the reaction was completed was added to each well of a 96-well fluorescence assay plate, and 75 μL of the Thioflavin-T solution prepared above was added to each well. After reacting for 5 minutes in the dark at room temperature, fluorescence values were measured at excitation 450 nm and emission 485 nm by using a multi-mode microplate reader.

The fluorescence value of the group (control) treated with Aβ alone and aggregated for 72 hours by treating only Aβ was considered as 100%, and the measured fluorescence values were expressed as a relative value thereto, and results thereof are shown in FIG. 7.

FIG. 7 shows the Aβ disaggregation effect of rifamycin-based derivatives, including rifapentine (control: a group treated with 50 μM $Aβ_{1-42}$ and aggregated for 3 or 5 days; test groups: 50 μM $Aβ_{1-42}$ was aggregated for 3 days, and treated with 50 μM rifamycin-based derivatives, followed by 2 days of reaction), wherein the degree of Aβ aggregation during rifapentine treatment was about 10% or less compared to the control, that is, rifapentine exhibited an Aβ disaggregation effect of about 90% or more compared to the control. In other words, it can be said that rifapentine shows better Aβ disaggregation effects than those of other derivatives (when treated with rifampicin, ripabutin, rifamycin SV, rifamdin, etc. the Aβ disaggregation effect was about 50% compared to the control, when treated with rifamycin S, the Aβ disaggregation effect was 25% disaggregation compared to the control, and when treated with rifaximin and rifamycin B, significant disaggregation did not occur compared to the control).

**Example 4: Confirmation of amyloid plaque reduction effect obtained by orally administered rifapentine in APP/PS1 TG mice**

**4.1. Preparing APP/PS1 TG Mouse Model**

Transgenic mice (APP/PS1 TG; Alzheimer' disease animal model; B6C3-Tg(APPswe,PSEN1dE)85Dbo/Mmjax) and wild type mice (WT) were derived from Jackson Laboratory (Bar Harbor, Maine, USA). APP/PS1 TG mice crossed with wild-type mice and maintained as double hemizygotes. All genotypes were confirmed by PCR analysis using tail DNA according to the standard PCR conditions of the Jackson Laboratory. Each mouse was housed in a plastic cage in the animal cage and the temperature of the plastic cage was maintained at 21±1 °C in alternating 12 hour light cycle, and the mice were freely fed with food and water.

**4.2. Rifapentine treatment in a mouse model**

Rifapentine was orally administered to 10-month-old APP/PS1 TG mice once per week (TG, RP 200 QW) at a concentration of 200 mg/kg or once every two days at a concentration of 50 mg/kg (TG, RP 50 QOD), for 9 weeks. The control received the same amount of vehicle (Veh) (0.5% MC in DW). Rifapentine was dissolved in 10 ml/kg (v/w) of the medium according to the body weight of the mouse and then administered through zonde for oral administration.

4.3. Preparation of brain tissue sample

Mice were anesthetized using 2% avertin (20 mg/g, i.p.). The mice were perfused with 0.9% NaCl and brains thereof were excised. The hemibrains were fixed overnight at 4 °C in 4% paraformaldehyde (pH 7.4). Paraformaldehyde-fixed brains were dehydrated to prepare tissue blocks using paraffin.

**4.4. Immunohistochemical staining**

Paraffin-immobilized brain tissues prepared in Example 4.3 were prepared in sections with a thickness of 5 to 6 μm, and immunohistochemical staining was performed thereon. Tissue sections were treated with dewax, rehydrate, and 1% hydrogen peroxide to remove endogenous peroxidase, followed by 60 minutes of blocking with 10% (v/v) goat normal serum to inhibit nonspecific reactions. Then, plaques were stained by using Aβ antibody (overnight, 4 °C). After washing with PBS the next day, the secondary antibody was stained using ABC kit (Vectastin ABC kit, Vector Laboratories) and then observed under a microscope. As shown in the staining image of Aβ plaques of FIG. 8 and the graph of FIGS. 9 and 10, compared to the control mice (TG-Veh), the number of plaques and the total area of plaques were reduced in rifapentine-treated mice.

**Example 5: Reduction of amyloid plaques and phosphorylated Tau by oral administration of triflupromazine in 5X FAD mice**

**5.1. Preparation of 5X FAD Mouse Model**

Transgenic mice (5XFAD; Alzheimer's disease animal model; B6SJL-Tg (APPSwFlLon, PSEN1*M146L*L286V)6799Vas/Mmjax)) and wild-type mice were obtained from Jackson Laboratory (Bar Harbor, Maine, USA). 5X FAD mice crossed with wild-type mice and maintained as double hemizygotes. All genotypes were confirmed by PCR analysis using tail DNA according to the standard PCR conditions of the Jackson Laboratory. Four to six mice were housed in a plastic cage in the animal cage and the temperature of the plastic cage was maintained at 21±1 °C in alternating 12 hour light cycle, and the mice were freely fed with food and water.

**5.2. Triflupromazine Treatment in a Mouse Model**

Seven-month old 5X FAD mice were orally treated with 50 mg/kg of triflupromazine once every two days for 3 weeks. The control received the same amount of vehicle (1% (w/v) DMSO in PBS). Rifapentine was dissolved in 10 ml/kg (v/w) of the vehicle according to the body weight of the mouse and then administered through zonde for oral administration.

**5.3. Preparation of brain tissue sample**

Mice were anesthetized using 2%(w/v) avertin (20 mg/g, i.p.). The mice were perfused with 0.9%(w/v) NaCl and brains thereof were excised. The hemibrains were fixed overnight at 4 °C in 4%(w/v) paraformaldehyde (pH 7.4). For cryopreservation, brains were soaked in 30%(w/v) sucrose for 48 hours. The other hemibrains were dissected into cortical and hippocampal regions and homogenized on ice for 30 minutes in lysis buffer (10 mM Tris-HCl, 5 mM EDTA in 320 mM sucrose, pH 7.4) containing 1x protease inhibitor cocktail. The sample was centrifuged at 13500 rpm for 30 minutes at 4 °C. The lysate concentration was measured using the BCA protein assay kit (thermoFisher, cat # 232250) according to the manufacturer's instructions. The absorbance was read at 560 nm.

**5.4. Immunohistochemistry Staining and ThS Staining**

To stain frozen brain with thioflavin S (ThS), the brain was cut to 35 um thickness to create tissue sections using cryostat (Leica CM1800) and then Aβ plaques were stained. ThS staining was performed for 7 minutes at a concentration of 500 uM in ethanol (50% (v/v)). The sections were then sequentially rinsed in 100, 90 and 70% (v/v)

ethanol for 10 seconds and placed in PBS. Images were obtained using a Leica DM2500 fluorescence microscope. As shown in FIG. 11, comparing the control mice (vehicle, 1% (w/v) DMSO in PBS) with the mice administered with triflupromazine according to the present disclosure, the number of plaques and total areas of plaques area were reduced in the mice administered triflupromazine.

### 5.5. Western blot assay method

Changes in Tau, a major cause of Alzheimer's dementia and Parkinson's disease, together with Aβ plaques, were confirmed by Western blot analysis. Samples of the prepared tissues were separated by SDS-PAGE and transferred to PVDF membrane. The membrane was treated overnight at 4 °C using the primary antibody. As the primary antibody, antibodies capable of detecting Total Tau (T-Tau) and phosphorylated Tau (P-Tau) were used, and detection was performed using a secondary antibody conjugated with horseradish peroxidase (HRP). The blot development was performed with a Clarity Western ECL substrate (Bio-Rad) according to the manufacturer's instructions.

The obtained Western blot results are shown in FIG. 12. As shown in FIG. 12, the control mice (Tg veh, 1% (w/v) DMSO in PBS) and the triflupromazine-administered mice (Tg TFP, n=4) were compared, and it was found that, in the case of the mice treated with triflupromazine, T-Tau (Total Tau) and P-Tau (phosphorylated Tau) were decreased in both the cortex and hippocampus tissue. These results show that triflupromazine lowers total Tau protein levels and inhibits phosphorylation of Tau proteins in transgenic animals (Tg) which are Alzheimer's disease animal model. On the other hand, in wild type mice (Wt), a decrease in total Tau (T-Tau) and phosphorylated Tau (P-Tau) was not observed.

## Example 6: Confirmation of increased cognitive function effect obtained by orally administered rifapentine in APP/PS1 TG mice

### 6.1. Preparing APP/PS1 TG Mouse Model

Transgenic mice (APP/PS1 TG; Alzheimer' disease animal model; B6C3-Tg(APPswe, PSEN1dE)85Dbo/Mmjax) and wild type mice were derived from Jackson Laboratory (Bar Harbor, Maine, USA). APP/PS1 TG mice crossed with wild-type mice and maintained as double hemizygotes. All genotypes were confirmed by PCR analysis using tail DNA according to the standard PCR conditions of the Jackson Laboratory. Each mouse was housed in a plastic cage in the animal cage and the temperature of the plastic cage was maintained at 21±1 °C in alternating 12 hour light cycle, and the mice were freely fed with food and water.

### 6.2. Rifapentine treatment in a mouse model

Rifapentine was orally administered to 10-month-old APP/PS1 TG mice once per week at a concentration of 200 mg/kg or once every two days at a concentration of 50 mg/kg, for 9 weeks. The control group received the same amount of vehicle (0.5% MC in DW). Rifapentine was dissolved in 10 ml/kg (v/w) of the medium according to the body weight of the mouse and then administered through zonde for oral administration.

### 6.3. Y-maze test

Y-maze test was performed using a maze structure made by placing the same three arms 40 cm in length (15 cm in height of the wall) at an angle of 120 degrees. This experiment was a behavioral experiment using rodent's instinctive search habits and focused on the possibility of exploring new areas. The more the test animal remembered the last arm it searched for and did not enter the same arm, the higher your memory was. The search time was 8 minutes per subject, and the final result was expressed as a spontaneous alteration (%) value. Spontaneous alteration (%) value was calculated by the following formula.

[Equation]

$$\text{Spontaneous alteration (\%)} = \text{The number of triplet}/(\text{total arm entry}-2)$$

Behavioral patterns were analyzed using SMART VIDEO TRACKING Software (Panlab, USA). All data obtained in the experiments were expressed as mean ± standard error mean (SEM), and the drug-applied group was compared with the non-drug group, which was a control, by using student's t-test. As shown in FIG. 13, on the $8^{th}$ week after rifapentine was administered to transgenic Alzheimer's dementia animals (APP PS1 TG), the Y-maze test was performed and it was found that the spontaneous alteration (%)value, which is a relative frequency of entering the maze sequentially

by identifying clues around, was increased closer to the positive control group (WT-Veh) compared to the negative control group (TG-Veh).

### 6.4. Novel Object Recognition Test

The novel object recognition test was performed to evaluate memory by using two different objects in a 40 cm x 40 cm acrylic cage. After accustoming the inside of the acrylic cage, two objects were placed at a constant position and the subjects were allowed to recognize the objects freely, and then the time for searching each object was measured. A 24-hour delay was given for each subject, and only one object was changed to another at the same location. At this time, when the changed object was recognized as a new object and thus the longer search time occurred, the corresponding memory was evaluated to be a good memory. When the subject does not remember objects that are placed 24 hours before, the subject can't distinguish between new and old objects, and may search both objects evenly. A total of 10 minutes were allowed to explore freely, and the result was expressed as a preference index (Novel object exploring time/total exploring time). The analysis was performed using SMART VIDEO TRACKING Software (Panlab, USA). All data obtained in the experiments were expressed as mean ± standard error mean (SEM), and the drug-applied group was compared with the non-drug group, which was a control, by using a student's t-test. As shown in FIG. 14, the results of novel object recognition test performed 8 weeks after rifapentine was administered to the transgenic Alzheimer's dementia animal (APP/PS1 TG) show that the value indicating a preference Index corresponding to the frequency at which an animal searches for new objects, was increased and the memory level was increased closer to the positive control group (WT-Veh) than to the negative control group (TG-Veh).

## Claims

1. A pharmaceutical composition for use in preventing or treating neurodegenerative brain disease, the pharmaceutical composition comprising rifapentine, or pharmaceutically acceptable salts thereof, as an active ingredient.

2. The pharmaceutical composition for use of claim 1, wherein the the pharmaceutical composition has an action of disaggregating amyloid-beta plaques.

3. The pharmaceutical composition for use of claim 1, wherein the pharmaceutical composition is administered to a subject selected from:

   (1) patients who have a higher level or a higher risk of amyloid-beta aggregation than normal individuals that do not have neurodegenerative brain disease;
   (2) patients who have a higher level or a higher risk of Tau protein aggregation than normal individuals that do not have neurodegenerative brain disease;
   (3) patients who have a higher level or a higher risk of Tau protein phosphorylation than normal individuals that do not have neurodegenerative brain disease; and
   (4) a patient who corresponds to one or more of (1) to (3) above.

4. The pharmaceutical composition for use of claim 1, wherein the neurodegenerative brain disease is selected from Alzheimer's disease, Parkinson's disease, Huntington's disease, mild cognitive impairment, cerebral amyloid angiopathy, down syndrome, amyloid stroke, systemic amyloid disease, Dutch-type amyloidosis, Niemann-Pick disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

5. A health functional food for use in preventing or treating neurodegenerative brain disease, the health functional food comprising rifapentine.

6. The health functional food for use of claim 5, wherein the health functional food has an action of disaggregating amyloid-beta plaques.

7. A health functional food for use in preventing or ameliorating cognitive disorders or for the therapeutic use in improving memory, the health functional food comprising rifapentine.

8. The health functional food for use of claim 7, wherein the health functional food has an action of disaggregating

amyloid-beta plaques.

## Patentansprüche

1. Arzneimittel zur Verwendung bei der Vorbeugung oder Behandlung einer neurodegenerativen Gehirnerkrankung, wobei das Arzneimittel Rifapentin oder pharmazeutisch verträgliche Salze davon als einen Wirkstoff umfasst.

2. Arzneimittel zur Verwendung nach Anspruch 1, wobei das Arzneimittel eine Wirkung der Desaggregation von Amyloid-beta-Plaques hat.

3. Arzneimittel zur Verwendung nach Anspruch 1, wobei das Arzneimittel einem Individuum verabreicht wird, das ausgewählt ist aus:

   (1) Patienten, die einen höheren Grad an oder ein höheres Risiko für Amyloid-beta-Ablagerung als normale Individuen aufweisen, die keine neurodegenerative Gehirnerkrankung haben;
   (2) Patienten, die einen höheren Grad an oder ein höheres Risiko für Tau-Protein-Ablagerung als normale Individuen aufweisen, die keine neurodegenerative Gehirnerkrankung haben;
   (3) Patienten, die einen höheren Grad an oder ein höheres Risiko für Tau-Protein-Phosphorylierung als normale Individuen aufweisen, die keine neurodegenerative Gehirnerkrankung haben; und
   (4) einem Patienten, der einem oder mehreren von (1) bis (3) oben entspricht.

4. Arzneimittel zur Verwendung nach Anspruch 1, wobei die neurodegenerative Gehirnerkrankung ausgewählt ist aus Alzheimer-Krankheit, Morbus Parkinson, Chorea Huntington, leichter kognitiver Beeinträchtigung, zerebraler Amyloidangiopathie, Down-Syndrom, Amyloid-Schlaganfall, systemischer Amyloidose, Amyloidose vom Holländischen Typ, Niemann-Pick-Syndrom, seniler Demenz, amyotropher Lateralsklerose, spinozerebellärer Atrophie, Tourette-Syndrom, Friedreich-Ataxie, Machado-Joseph-Krankheit, Lewy-Körperchen-Demenz, Dystonie, progressiver supranukleärer Blickparese und frontotemporaler Demenz.

5. Gesundheitsförderndes funktionelles Lebensmittel zur Verwendung bei der Vorbeugung oder Behandlung neurodegenerativer Gehirnerkrankung, wobei das gesundheitsfördernde funktionelle Lebensmittel Rifapentin umfasst.

6. Gesundheitsförderndes funktionelles Lebensmittel zur Verwendung nach Anspruch 5, wobei das gesundheitsfördernde funktionelle Lebensmittel eine Wirkung der Desaggregation von Amyloid-beta-Plaques hat.

7. Gesundheitsförderndes funktionelles Lebensmittel zur Verwendung bei der Vorbeugung oder Besserung kognitiver Störungen oder zur therapeutischen Verwendung bei der Verbesserung des Gedächtnisses, wobei das gesundheitsfördernde funktionelle Lebensmittel Rifapentin umfasst.

8. Gesundheitsförderndes funktionelles zur Verwendung nach Anspruch 7, wobei das gesundheitsfördernde funktionelle Lebensmittel eine Wirkung der Desaggregation von Amyloid-beta-Plaques hat.

## Revendications

1. Composition pharmaceutique pour une utilisation dans la prévention ou le traitement d’une maladie cérébrale neurodégénérative, la composition pharmaceutique comprenant de la rifapentine, ou des sels pharmaceutiquement acceptables correspondants, en tant qu’ingrédient actif.

2. Composition pharmaceutique pour une utilisation selon la revendication 1, la composition pharmaceutique ayant une action de désagrégation de plaques amyloïdes-bêta.

3. Composition pharmaceutique pour une utilisation selon la revendication 1, la composition pharmaceutique étant administrée à un sujet choisi parmi :

   (1) des patients présentant un taux plus élevé ou un risque plus élevé d’agrégation d’amyloïde-bêta que des individus normaux ne présentent pas de maladie cérébrale neurodégénérative ;
   (2) des patients présentant un taux plus élevé ou un risque plus élevé d’agrégation de protéine Tau que des

individus normaux ne présentent pas de maladie cérébrale neurodégénérative ;
(3) des patients présentant un taux plus élevé ou un risque plus élevé de phosphorylation de protéine Tau que des individus normaux ne présentent pas de maladie cérébrale neurodégénérative ; et
(4) un patient qui correspond à l'un ou plusieurs parmi (1) à (3) ci-dessus.

**4.** Composition pharmaceutique pour une utilisation selon la revendication 1, la maladie cérébrale neurodégénérative étant choisie parmi la maladie d'Alzheimer, la maladie de Parkinson, la maladie de Huntington, une déficience cognitive légère, une angiopathie amyloïde cérébrale, le syndrome de Down, un accident vasculaire cérébral amyloïde, une maladie amyloïde systémique, une amyloïdose de type Dutch, la maladie de Niemann-Pick, la démence sénile, la sclérose latérale amyotrophique, une atrophie spinocérebelleuse, le syndrome de Gilles de la Tourette, l'ataxie de Friedrich, la maladie de Machado-Joseph, la démence à corps de Lewy, la paralysie supranucléaire progressive, et la démence frontotemporale.

**5.** Aliment fonctionnel de santé pour une utilisation dans la prévention ou le traitement d'une maladie cérébrale neurodégénérative, l'aliment fonctionnel de santé comprenant de la rifapentine.

**6.** Aliment fonctionnel de santé pour une utilisation selon la revendication 5, l'aliment fonctionnel de santé ayant une action de désagrégation de plaques amyloïdes-bêta.

**7.** Aliment fonctionnel de santé pour une utilisation dans la prévention ou l'amélioration de troubles cognitifs ou pour l'utilisation thérapeutique dans l'amélioration de la mémoire, l'aliment fonctionnel de santé comprenant de la rifapentine.

**8.** Aliment fonctionnel de santé pour une utilisation selon la revendication 7, l'aliment fonctionnel de santé ayant une action de désagrégation de plaques amyloïdes-bêta.

Fig.1

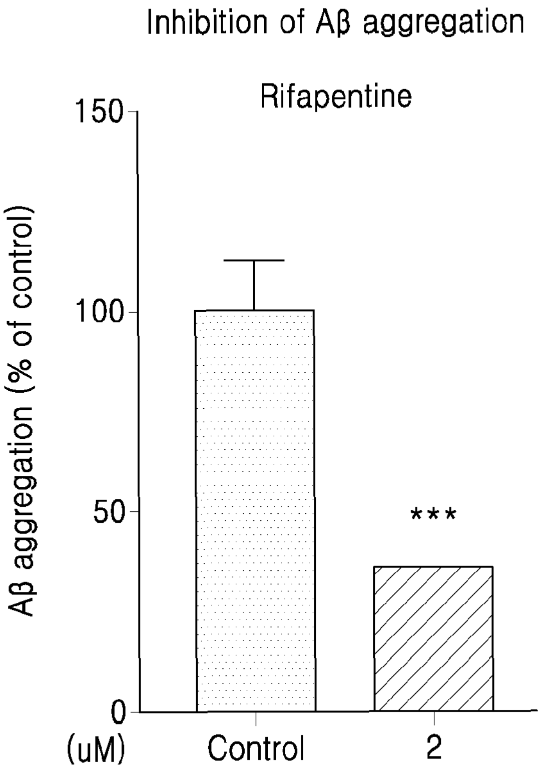
Inhibition of Aβ aggregation
Rifapentine
Aβ aggregation (% of control)
150
100
50
0
***
(uM)
Control
2

Fig.2

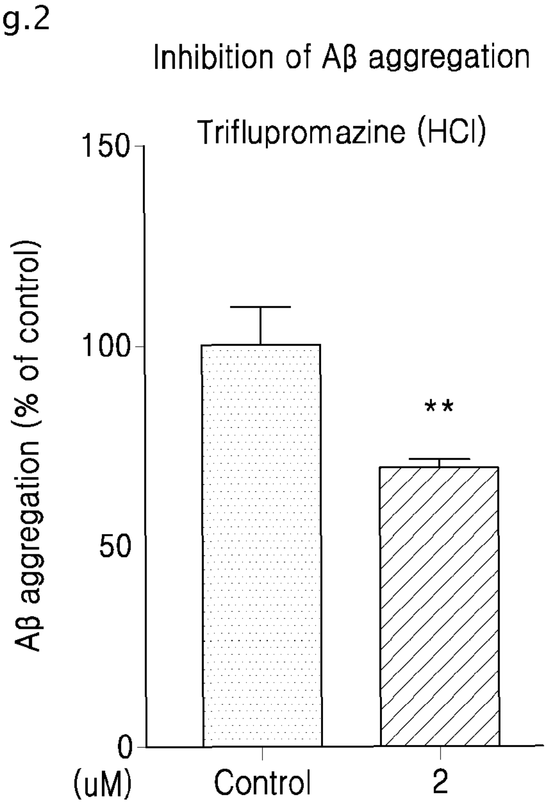
Inhibition of Aβ aggregation
Triflupromazine (HCl)
Aβ aggregation (% of control)
150
100
50
0
**
(uM)
Control
2

Fig.3

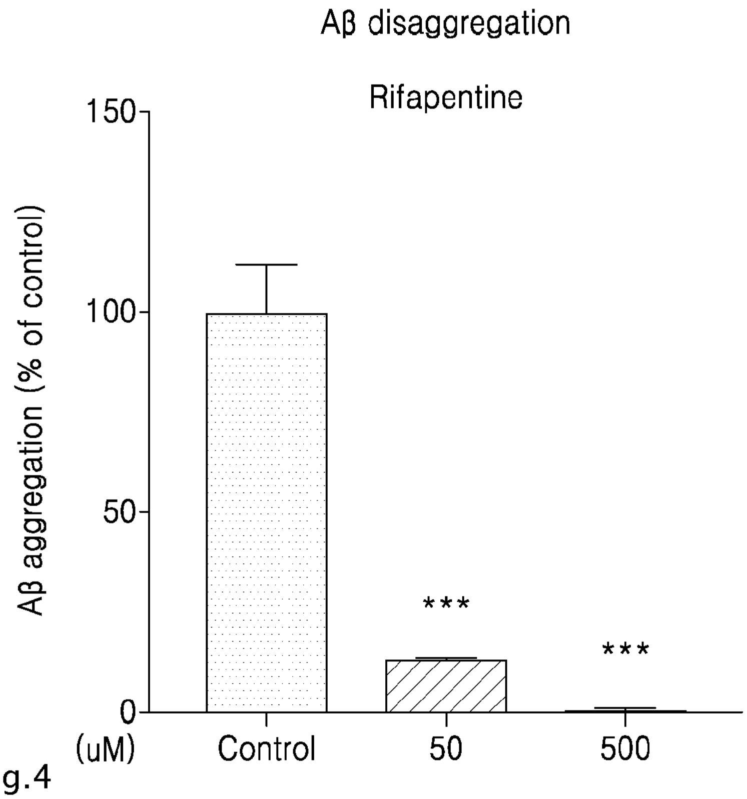
Aβ disaggregation
Rifapentine
150
100
50
0
Aβ aggregation (% of control)
***
***
(uM)
Control
50
500

Fig.4

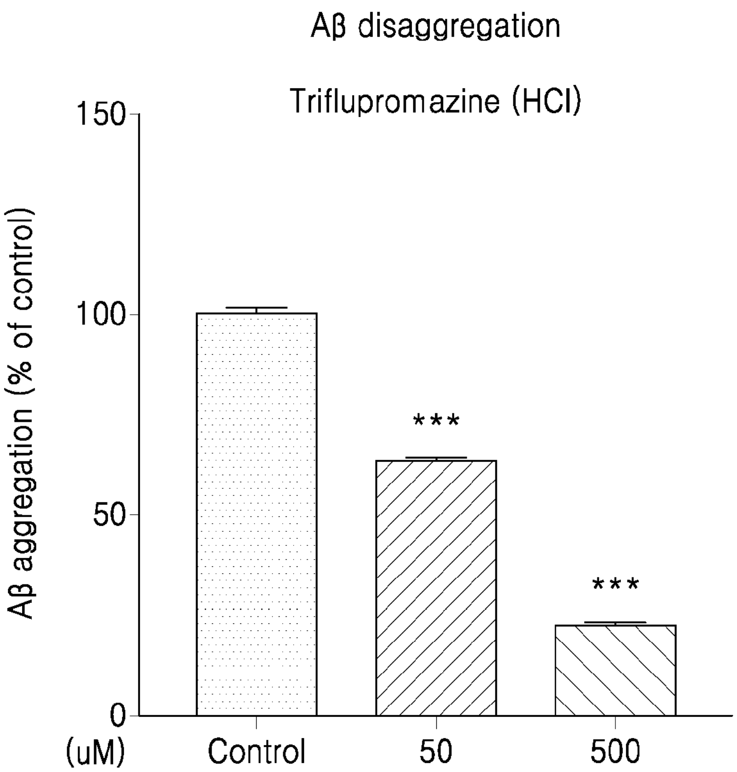
Aβ disaggregation
Triflupromazine (HCl)
150
100
50
0
Aβ aggregation (% of control)
***
***
(uM)
Control
50
500

Fig.5

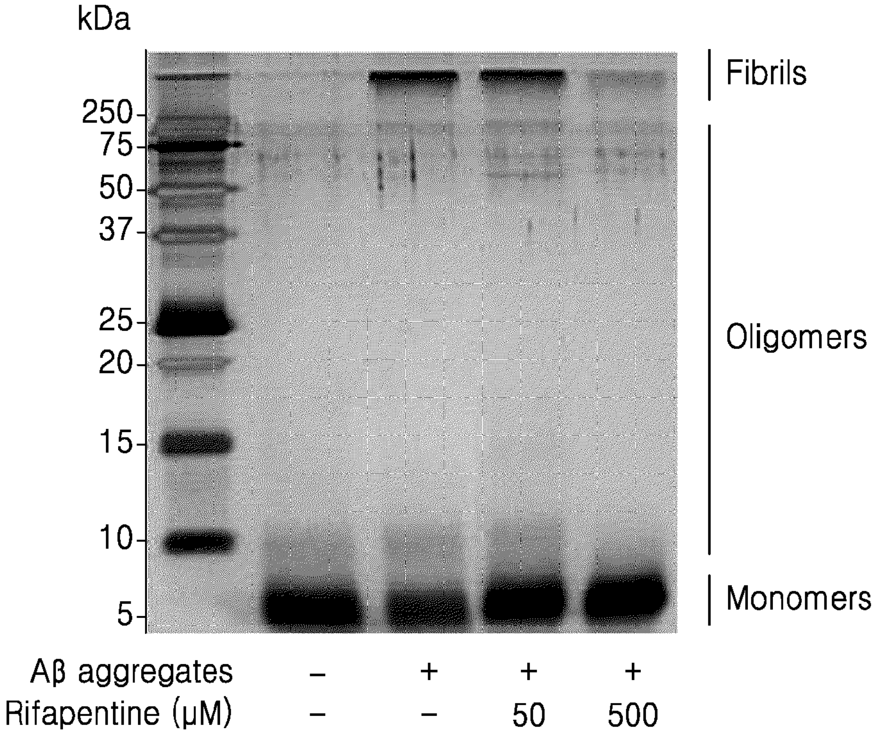
kDa
250
75
50
37
25
20
15
10
5
Fibrils
Oligomers
Monomers
Aβ aggregates – + + +
Rifapentine (μM) – – 50 500

Fig.6

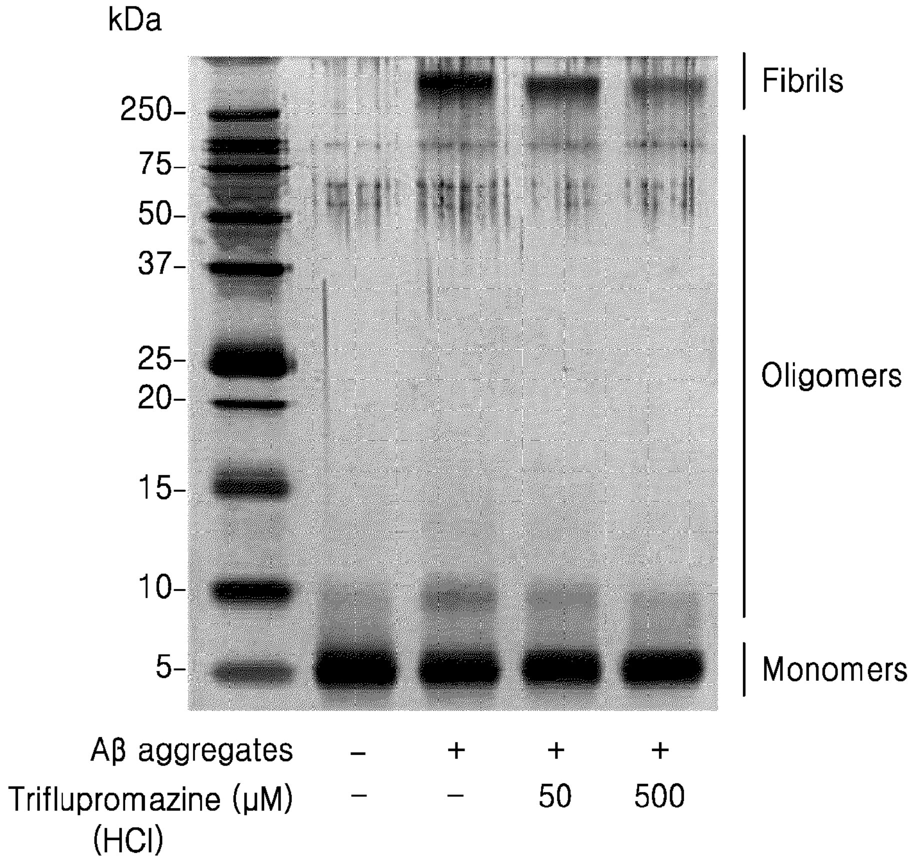
kDa
250
75
50
37
25
20
15
10
5
Fibrils
Oligomers
Monomers
Aβ aggregates – + + +
Triflupromazine (μM) – – 50 500
(HCl)

Fig.7

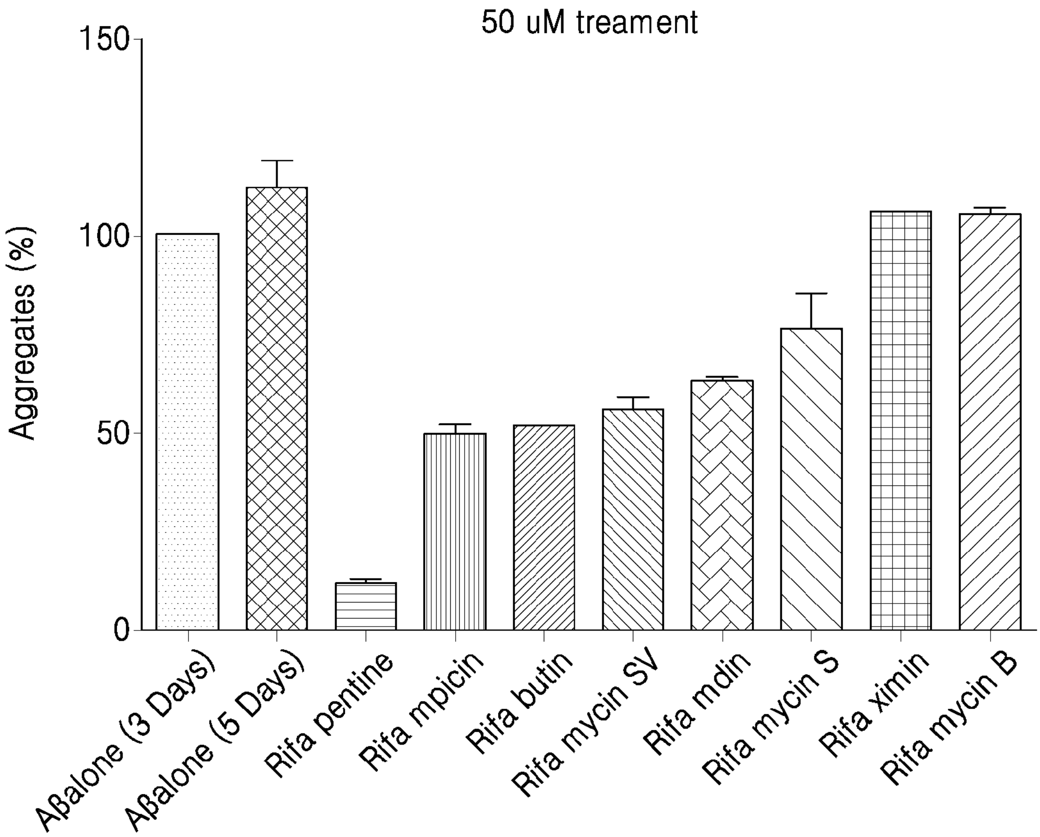
50 uM treament
Aggregates (%)
150
100
50
0
Aβalone (3 Days)
Aβalone (5 Days)
Rifa pentine
Rifa mpicin
Rifa butin
Rifa mycin SV
Rifa mdin
Rifa mycin S
Rifa ximin
Rifa mycin B

Fig.8

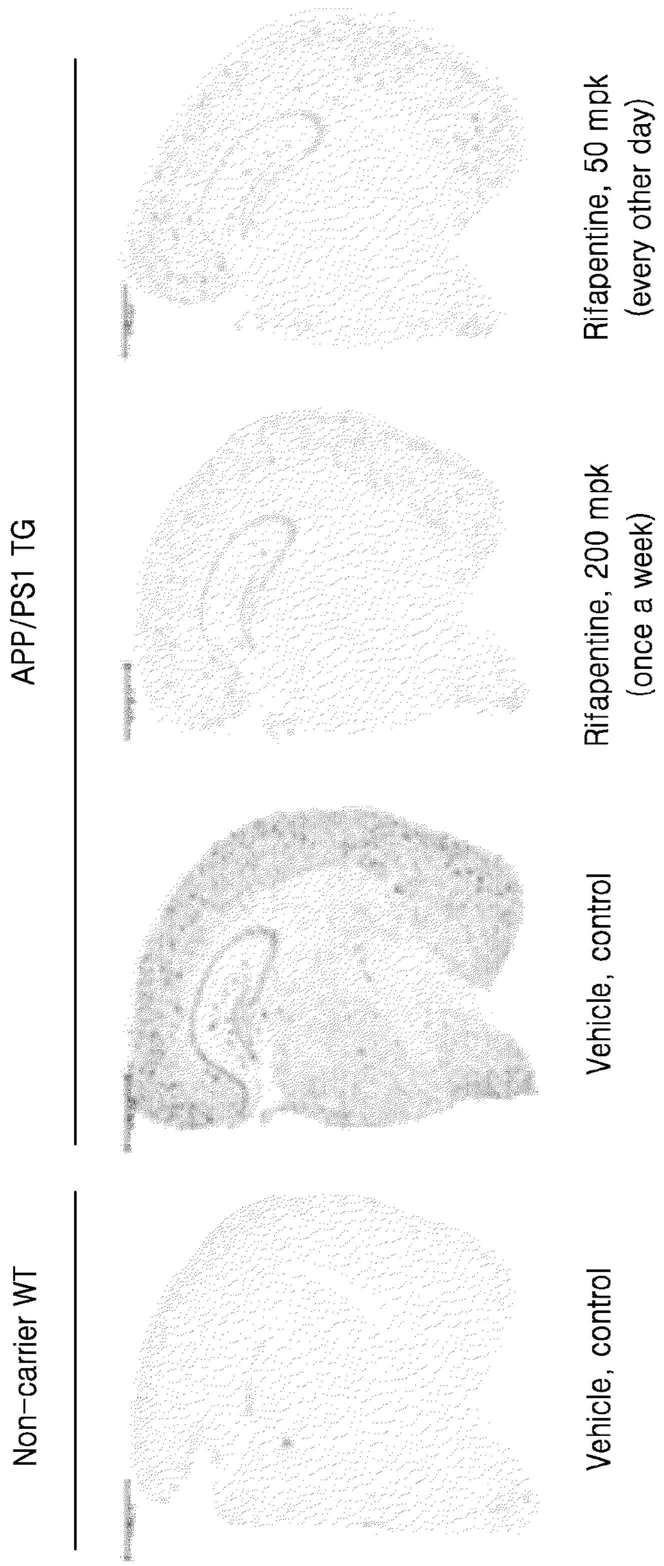
Non-carrier WT
APP/PS1 TG
Vehicle, control
Vehicle, control
Rifapentine, 200 mpk
(once a week)
Rifapentine, 50 mpk
(every other day)

Fig.9

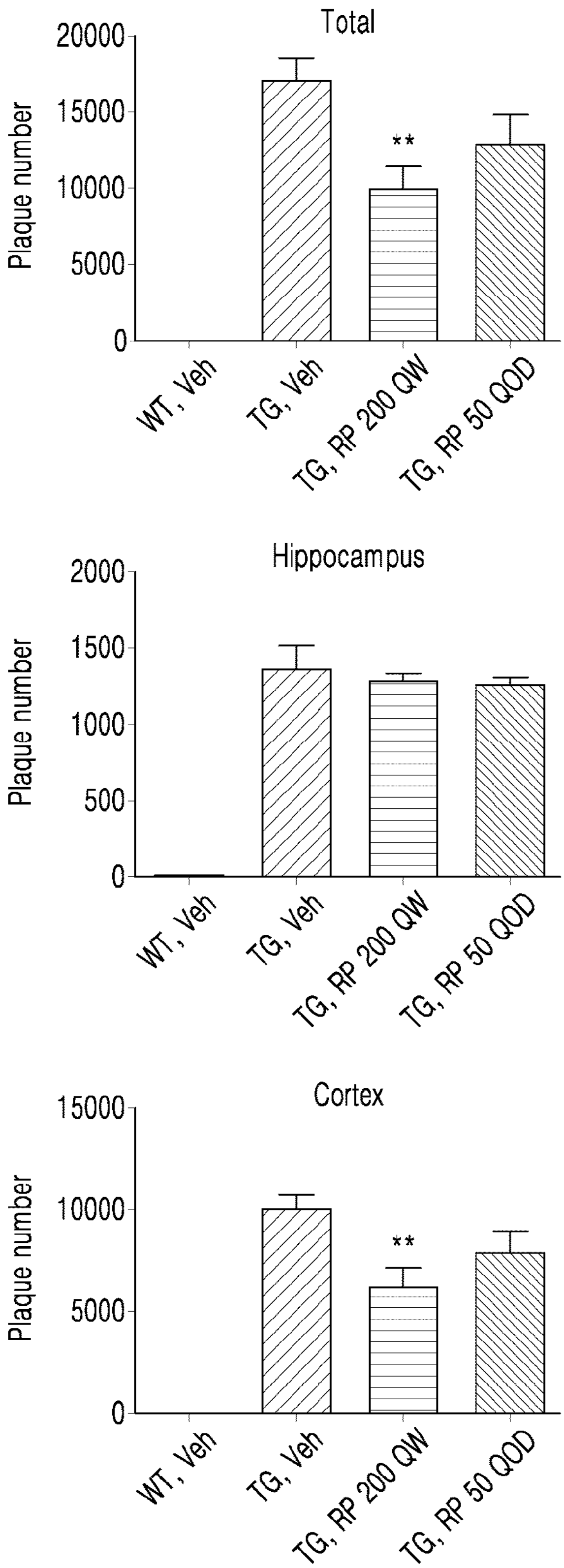
Total
Plaque number
20000
15000
10000
5000
0
**
WT, Veh
TG, Veh
TG, RP 200 QW
TG, RP 50 QOD
Hippocampus
Plaque number
2000
1500
1000
500
0
WT, Veh
TG, Veh
TG, RP 200 QW
TG, RP 50 QOD
Cortex
Plaque number
15000
10000
5000
0
**
WT, Veh
TG, Veh
TG, RP 200 QW
TG, RP 50 QOD

Fig.10

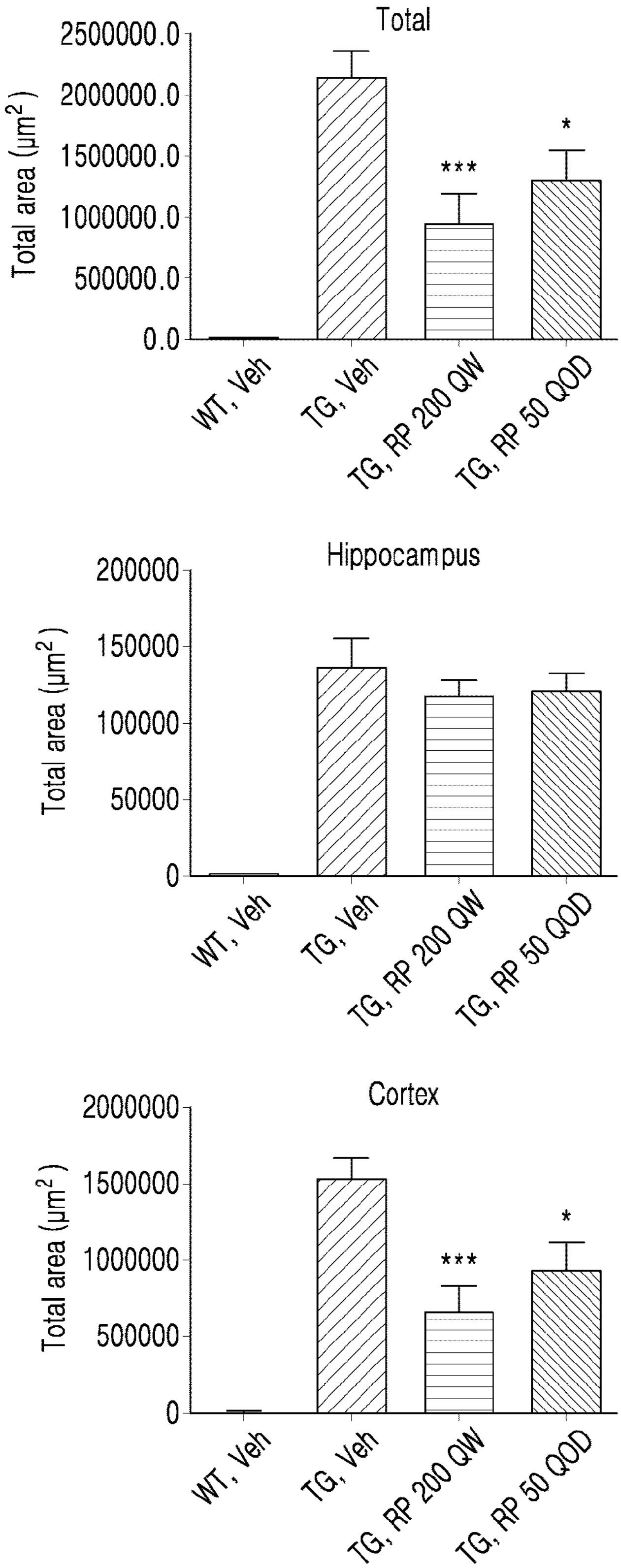
Total
Total area (μm2)
2500000.0
2000000.0
1500000.0
1000000.0
500000.0
0.0
***
*
WT, Veh
TG, Veh
TG, RP 200 QW
TG, RP 50 QOD
Hippocampus
Total area (μm2)
200000
150000
100000
50000
0
WT, Veh
TG, Veh
TG, RP 200 QW
TG, RP 50 QOD
Cortex
Total area (μm2)
2000000
1500000
1000000
500000
0
***
*
WT, Veh
TG, Veh
TG, RP 200 QW
TG, RP 50 QOD

Fig.11

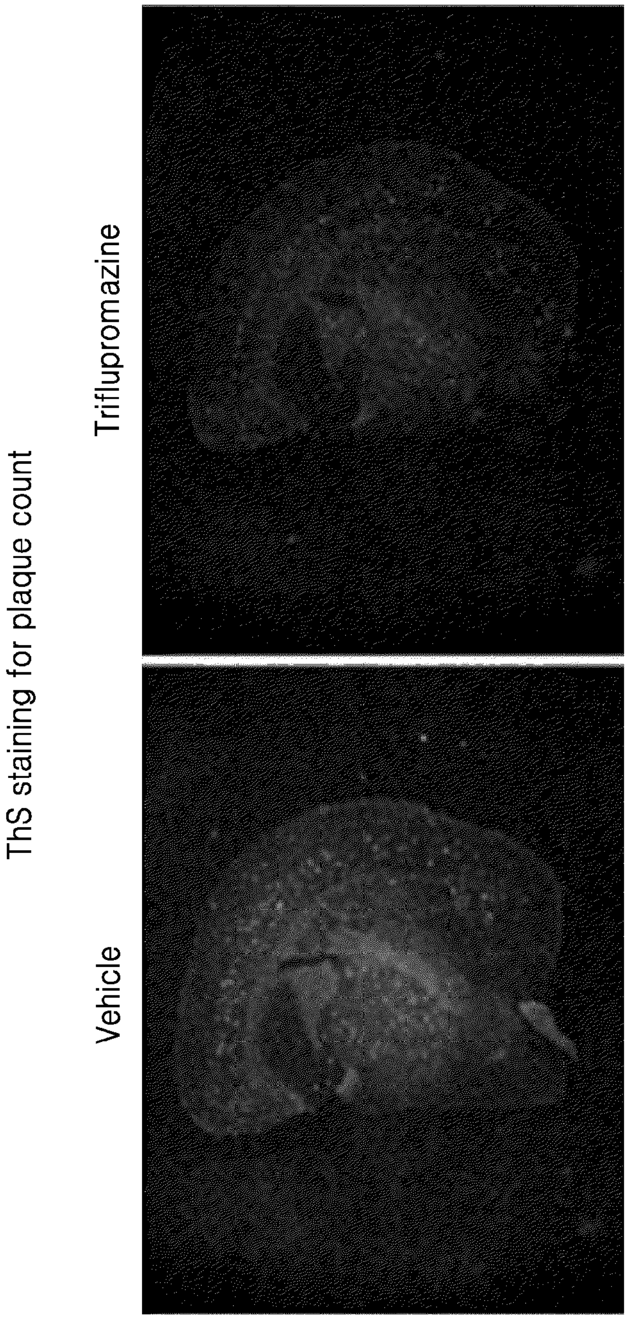

Fig.12

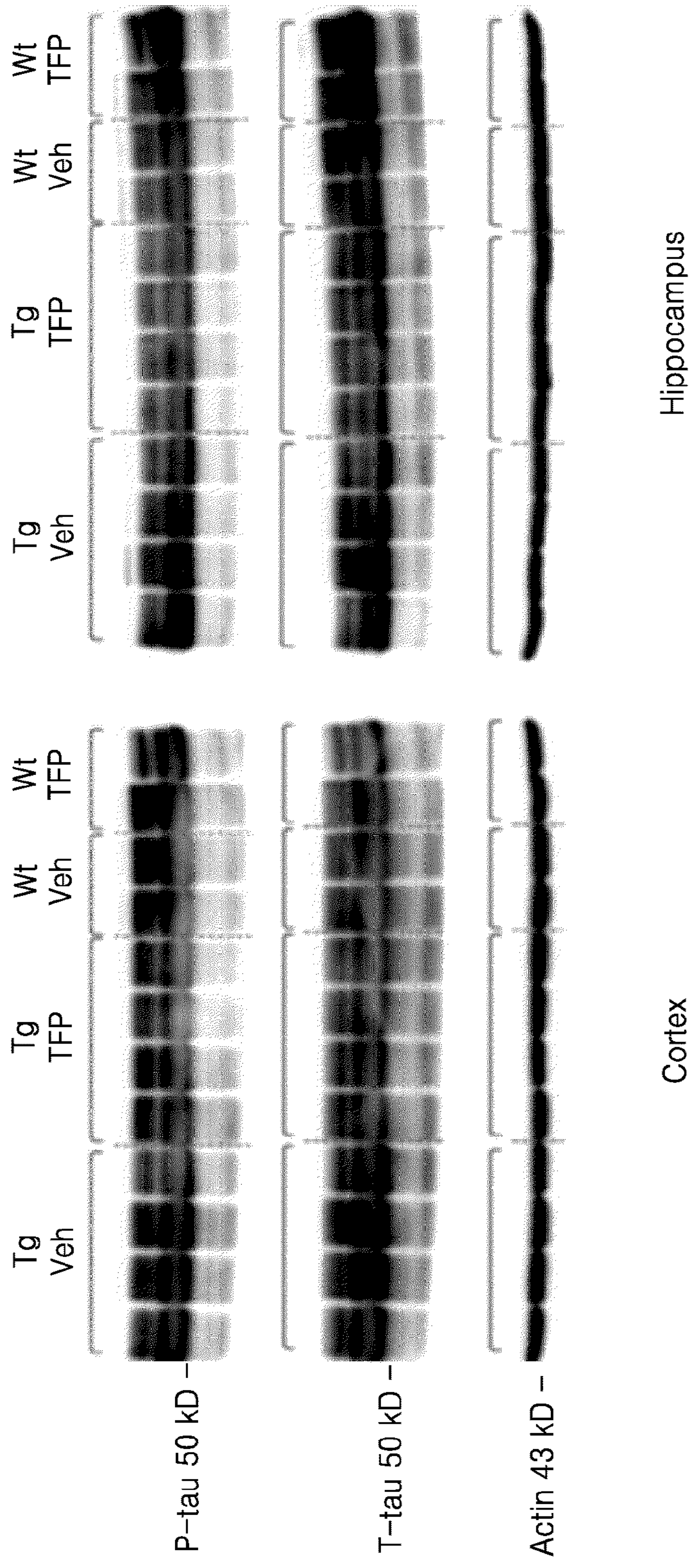
Wt TFP
Wt Veh
Tg TFP
Tg Veh
Hippocampus
Cortex
P–tau 50 kD –
T–tau 50 kD –
Actin 43 kD –

Fig.13

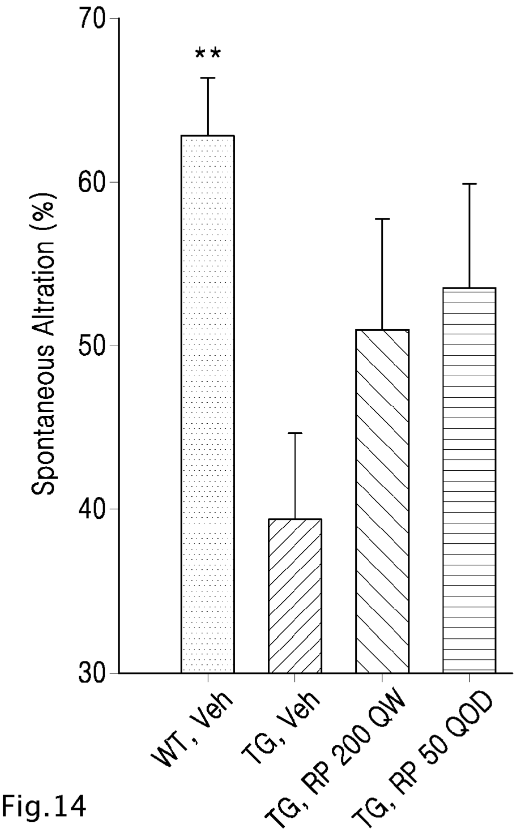
Spontaneous Altration (%)
70
60
50
40
30
**
WT, Veh
TG, Veh
TG, RP 200 QW
TG, RP 50 QOD

Fig.14

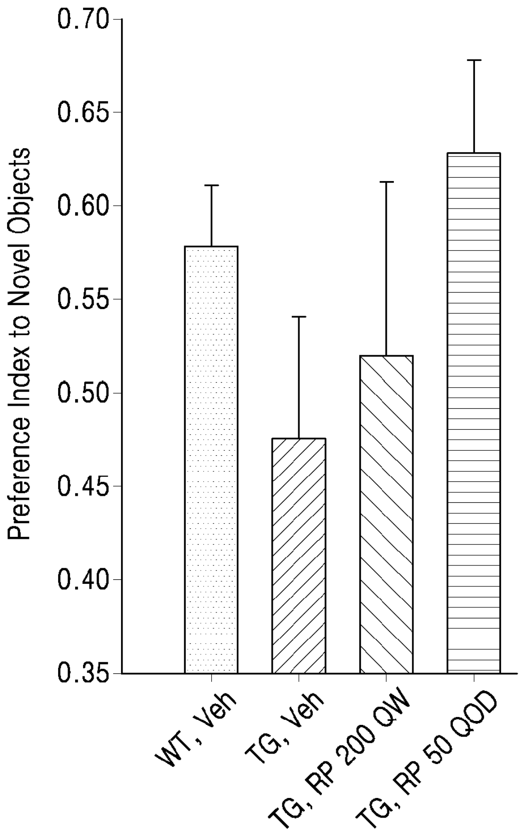
Preference Index to Novel Objects
0.70
0.65
0.60
0.55
0.50
0.45
0.40
0.35
WT, Veh
TG, Veh
TG, RP 200 QW
TG, RP 50 QOD

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0675126 A


### Non-patent literature cited in the description


- **MANDELKOW et al.** *Acta.Neuropathol.,* 1996, vol. 103, 26-35
- **TOMIYAMA et al.** *Biochem. Biophys. Res. Comm.,* 1994, vol. 204 (1), 76-83
- **UMEDA et al.** *Brain,* 2016, vol. 139 (5), 1568-1586
- **UMEDA et al.** *Alzheimer's & Dementia: Translational Research & Clinical Interventions,* 2018, vol. 4, 304-313
- **IZUKA et al.** *Dement. Geriatr. Cogn. Dis. Extra.,* 2017, vol. 7 (2), 204-214
- *CHEMICAL ABSTRACTS,* 146-54-3